# EUROPEAN PATENT APPLICATION

(11) **EP 4 545 520 A1**
(43) Date of publication of application: **30.04.2025**
(21) Application number: 23845472.2
(22) Date of filing: 24.07.2023
(51) Int. Cl.: C07D 405/04

(54) **SALT OF ENDOTHELIN A (ETA) RECEPTOR ANTAGONIST COMPOUND, AND PREPARATION METHOD THEREFOR AND MEDICAL USE THEREOF**

(30) Priority: 25.07.2022 CN 202210882770
(71) Applicant: Shenzhen Salubris Pharmaceuticals Co., Ltd, Shenzhen, Guangdong 518017 (CN)
(72) Inventor: WU, Junjun, Shenzhen, Guangdong 518017 (CN); LU, Yinsuo, Shenzhen, Guangdong 518017 (CN); LI, Song, Shenzhen, Guangdong 518017 (CN); XU, Wenjie, Shenzhen, Guangdong 518017 (CN); LI, Qinze, Shenzhen, Guangdong 518017 (CN); XU, Cheng, Shenzhen, Guangdong 518017 (CN); YU, Fangcai, Shenzhen, Guangdong 518017 (CN); HUA, Huaijie, Shenzhen, Guangdong 518017 (CN)
(74) Representative: Groth & Co. KB
(86) International application number: PCT/CN2023/108780
(87) International publication number: WO 2024/022262

(57) **Abstract**

The present invention belongs to the technical field of chemical drugs. Provided are a salt of an endothelin A (ETA) receptor antagonist compound, and a preparation method therefor and the use thereof.

## Description

### TECHNICAL FIELD

The present disclosure belongs to the technical field of chemical drugs, and provides a salt of an endothelin A (ETA) receptor antagonist compound, and a preparation method therefor and use thereof.

### BACKGROUND

Atrasentan (CAS: 173937-91-2) is an effective and selective endothelin A (ETA) receptor antagonist, with the following structural formula:

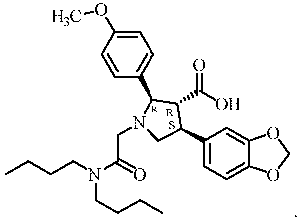

Applied clinically is Atrasentan hydrochloride (CAS: 195733-43-8), with the following structure:

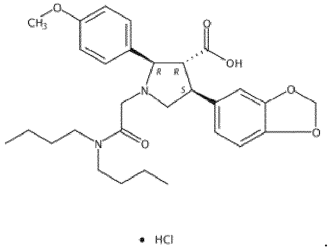

It was previously evaluated in clinical trials for the treatment of prostate cancer, but now it is evaluated in clinical trials for the treatment of chronic kidney disease associated with type II diabetes. At the same time, it has been proved that it can reduce albuminuria of patients with diabetic nephropathy, and there is no product on the market at present.

Nearly half of drug molecules existed in a form of salts, and meanwhile, salt formation can improve certain undesirable physical, chemical, or biological properties of the drug. Therefore, it is of great significance to develop a salt of an endothelin A (ETA) receptor antagonist compound with superior physicochemical or pharmaceutical properties.

### SUMMARY OF THE INVENTION

In view of problems existed in the prior art, the present disclosure provides a structurally-novel salt of an endothelin A (ETA) receptor antagonist compound, and a preparation method therefor and use thereof.

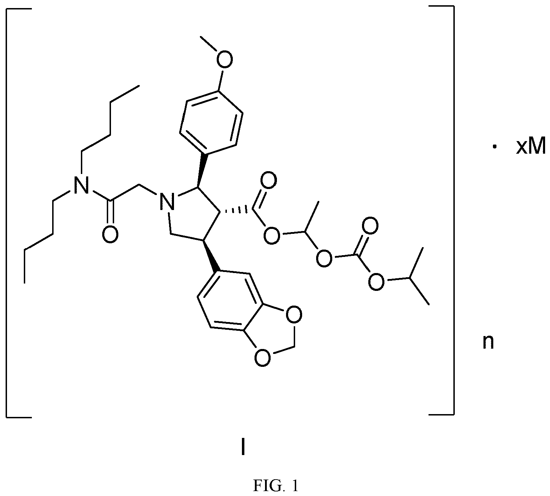

Specifically, the present disclosure provides a salt of a compound represented by formula (I), a structural formula of which is as shown in FIG. 1 below:

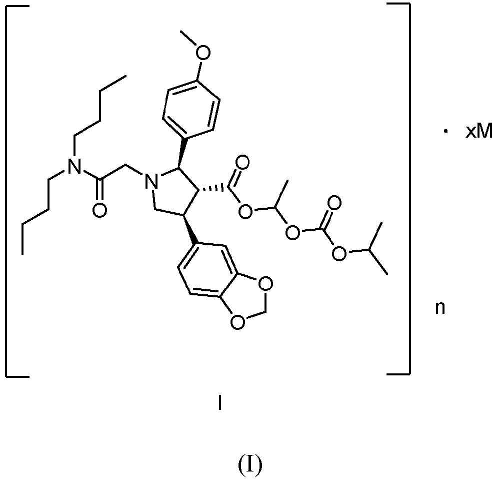

M is an inorganic acid or an organic acid, where n:x=1:(1-2).

As a preferred technical solution of the present disclosure, the inorganic acid is selected from the group consisting of sulfuric acid, hydrochloric acid, hydrofluoric acid, hydrobromic acid, hydroiodic acid, phosphoric acid, carbonic acid and nitric acid; and

the organic acid is selected from the group consisting of benzoic acid, 2,5-dihydroxybenzoic acid, 4-acetaminobenzoic acid, 4-aminobenzoic acid, oxalic acid, acetic acid, dichloroacetic acid, trichloroacetic acid, hexanoic acid, adipic acid, benzenesulfonic acid, 4-chlorobenzenesulfonic acid, octanoic acid, decanoic acid, cinnamic acid, citric acid, aspartate, gluconic acid, glutamic acid, lactic acid, malic acid, mandelic acid, pyroglutamic acid, tartaric acid, succinic acid, formic acid, fumaric acid, gentian acid, glutaric acid, pentanoic acid, aspartic acid, lauric acid, camphanic acid, maleic acid, propanedioic acid, nicotinic acid, propionic acid, salicylic acid, 4-aminosalicylic acid, decanedioic acid, stearic acid, butanedioic acid, palmitic acid, pamoic acid, trifluoroacetic acid, thiocyanic acid, p-toluenesulfonic acid, and L-malic acid.

As a preferred technical solution of the present disclosure, the inorganic acid is phosphoric acid.

As a preferred technical solution of the present disclosure, the inorganic acid is phosphoric acid, where n=3, x=3-6.

As a preferred technical solution of the present disclosure, the inorganic acid is phosphoric acid, where n=3, x=3; or n=3, x=4; or n=3, x=5; or n=3, x=6.

As a preferred technical solution of the present disclosure, the organic acid is oxalic acid.

As a preferred technical solution of the present disclosure, the organic acid is oxalic acid, where n=1, x=1.

The present disclosure further provides a pharmaceutical composition, and the pharmaceutical composition includes the salt of the compound represented by formula (I), and more than one pharmaceutically acceptable carrier.

The present disclosure further provides use of the salt of the compound represented by formula (I) in the manufacture of a medicament for treatment and/or prevention of a disease associated with endothelin A (ETA) receptor antagonism.

As a preferred technical solution of the present disclosure, the disease includes chronic kidney disease, IgA, FSGS, Alport, and hypertension.

The present disclosure further provides a method for preparing the salt of the compound represented by formula (I), wherein the salt of the compound is prepared by mixing a compound A with an acid.

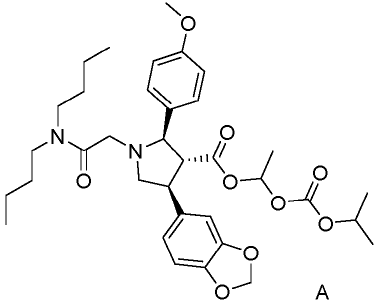

As a preferred technical solution of the present disclosure, a molar ratio of the compound to an acid molecule is 1:(1-2).

As a preferred technical solution of the present disclosure, the method includes the following steps:
1) weighing an appropriate amount of free alkali and dissolving the same with a benign solvent to obtain a solution;
2) weighing an appropriate amount of ionic acid and adding the same into the solution of step 1 for reaction to form a salt;
3) dripping a reaction solution from step 2 back into a poor solvent, stirring and precipitating; and
4) obtaining the salt of the compound by quickly centrifuging or standing.

The acid is M which is an inorganic acid or an organic acid, and the inorganic acid is selected from the group consisting of sulfuric acid, hydrochloric acid, hydrofluoric acid, hydrobromic acid, hydroiodic acid, phosphoric acid, carbonic acid and nitric acid. The organic acid is selected from the group consisting of benzoic acid, 2,5-dihydroxybenzoic acid, 4-acetaminobenzoic acid, 4-aminobenzoic acid, oxalic acid, acetic acid, dichloroacetic acid, trichloroacetic acid, hexanoic acid, adipic acid, benzenesulfonic acid, 4-chlorobenzenesulfonic acid, octanoic acid, decanoic acid, cinnamic acid, citric acid, aspartate, gluconic acid, glutamic acid, lactic acid, malic acid, mandelic acid, pyroglutamic acid, tartaric acid, succinic acid, formic acid, fumaric acid, gentian acid, glutaric acid, pentanoic acid, aspartic acid, lauric acid, camphanic acid, maleic acid, propanedioic acid, nicotinic acid, propionic acid, salicylic acid, 4-aminosalicylic acid, decanedioic acid, stearic acid, butanedioic acid, palmitic acid, pamoic acid, trifluoroacetic acid, thiocyanic acid, p-toluenesulfonic acid, and L-malic acid.

The solvent used for salt formation in the present disclosure is selected from at least one of ethyl acetate, methanol, n-propanol, isopropanol, isopropyl ether, tetrahydrofuran, isopropyl acetate, acetone, methyl tert-butyl ether, acetonitrile, ethanol, 1,4-dioxane, n-hexane, and isopropyl ether.

Further, in an optional implementation solution, the method for preparing the aforementioned pharmaceutically acceptable salt further includes steps such as evaporating the solvent or stirring for crystallization, filtering, and drying.

Unless otherwise specified, the following terms and phrases used herein are intended to have the following meanings. A specific term or phrase should not be considered uncertain or unclear without a specific definition, but should be understood according to ordinary meanings. When a commodity name appears herein, it is intended to refer to a commodity or an active ingredient corresponding to the commodity name. A term "pharmaceutically acceptable" used here refers to compounds, materials, compositions, and/or dosage forms that are suitable for use in contact with human and animal tissues within a range of reliable medical judgment, without excessive toxicity, irritation, allergic reactions, or other issues or complications, and are commensurate with a reasonable benefit/risk ratio.

A "pharmaceutically acceptable salt" used herein belong to derivatives of the compound of the present disclosure, wherein a parent compound is modified by forming a salt with an acid or an alkali.

A prodrug of the compound described herein easily undergoes chemical changes under a physiological condition, thereby being transformed into the compound of the present disclosure. In addition, the prodrug can be converted into the compound of the present disclosure through a chemical or biochemical method in an in vivo environment.

Certain compounds of the present disclosure may exist in a non-solvation or solvation form, including a hydrate form. Generally speaking, the solvation form and the non-solvation form are equivalent and are both contained within the scope of the present disclosure.

Atoms of molecules of the compound of the present disclosure are isotopes, and effects such as prolonging half-life, decreasing a clearance rate, enhancing metabolic stability, and improving in vivo activity can generally be achieved through isotope derivatization. Moreover, one implementation solution in which at least one atom is replaced by an atom with the same atom number (proton number) and different mass numbers (a sum of protons and neutrons) is included. Examples of the isotopes included in the compound of the present disclosure include hydrogen atoms, carbon atoms, nitrogen atoms, oxygen atoms, phosphorus atoms, sulfur atoms, fluorine atoms, and chlorine atoms, which respectively include ²H, ³H, ¹³C, ¹⁴C, ¹⁵ N , ¹⁷O, ¹⁸O, ³¹P, ³²P, ³⁵S, ¹⁸F, and ³⁶Cl. Particularly, radioactive isotopes such as 3H or 14C that emit radiation upon decaying can be used for local anatomical examination of pharmaceutic preparations or in vivo compounds. Stable isotopes neither decay or change with their quantity, nor have radioactivity, so the stable isotopes can be safely used. When the atoms constituting the molecules of the compound of the present disclosure are the isotopes, the isotopes can be transformed according to a general method by replacing a reagent used in synthesis with a reagent containing the corresponding isotopes.

The compound of the present disclosure may contain unnatural proportions of atomic isotopes on one or more atoms constituting the compound. For example, the compound may be labeled with the radioactive isotopes such as deuterium (²H), iodine-125 (¹²⁵I), or C-14 (¹⁴C). Transformations of all isotope compositions of the compound of the present disclosure, whether radioactive or not, are included within the scope of the present disclosure. Further, the compound of the present disclosure has one or more hydrogen atoms replaced by the isotope deuterium (²H). After deuteration, the compound of the present disclosure has the effects such as prolonging half-life, decreasing clearance rate, enhancing metabolic stability, and improving in vivo activity. A method for preparing isotope derivatives usually includes a phase transfer catalysis method. For example, a preferred deuteration method uses a phase transfer catalyst (such as tetraalkylammonium salt, NBu₄HSO₄). Using the phase transfer catalyst to exchange a methylene proton of a diphenylmethane compound results in introduction of a higher deuterium level compared to reducing with sodium deuterated borate using deuterated silane (such as triethyldeuterated silane) in the presence of an acid (such as methanesulfonic acid) or using a Lewis acid such as aluminum trichloride.

A term "pharmaceutically acceptable carrier" refers to any preparation carrier or medium capable of delivering an effective dosage of the active substance of the present disclosure, not interfering with biological activity of the active substance, and having no toxic side effects to a host or a patient. Representative carriers include water, oil, vegetables and minerals, cream bases, lotion bases, ointment bases, etc. These bases include suspension agents, thickening agents, transdermal enhancers, etc. Their preparations are well known to technicians in a cosmetics field or a topical drug field. For other information about the carrier, please refer to Remington: The Science and Practice of Pharmacy, 21st Ed., Lippincott, Williams & Wilkins (2005), of which the contents are incorporated herein by reference.

A term "excipient" typically refers to a carrier, a diluent, and/or a medium required for the preparation of an effective pharmaceutical composition.

For drugs or pharmacologically active agents, a term "effective dosage" or "therapeutic effective dosage" refers to a sufficient usage amount of drugs or medicament that is non-toxic but can achieve the desired effect. For an oral dosage form in the present disclosure, an "effective dosage" of one active substance in the composition refers to the usage amount required to achieve the desired effect when combined with another active substance in the composition. The determination of the effective dosage varies from person to person, depending on an age and general situation of a receptor, as well as the specific active substance. The appropriate effective dosage in each case may be determined by those skilled in the art according to routine experiments.

Terms "active ingredient", "therapeutic agent", "active substance" or "active agent" refer to a chemical entity that can effectively treat target disorders, diseases or conditions.

A term "tautomer" or "tautomeric form" refers to structural isomers with different energies that can be transformed into each other through a low energy barrier. If tautomerism is possible (such as in a solution), chemical equilibrium of the tautomer can be achieved. For example, a protontautomer (also known as prototropic tautomer) includes mutual transformation through proton transfer, such as ketone-enol isomerization and imine-enamine isomerization. A valence tautomer includes mutual transformation through recombination of some bonding electrons. Ketone-enol is in tautomerism. Another example of tautomerism is phenol-ketone tautomerism. Unless otherwise specified, all tautomer forms of the compound of the present disclosure are within the scope of the present disclosure.

The compound of the present disclosure may exist in a specific geometric or stereoisomeric form. The present disclosure envisions all such compounds, including cis and trans isomers, (-)- and (+)- enantiomers, (R)- and (S)- enantiomers, diastereomer, (D)-isomer, (L)- isomer, as well as racemic mixtures thereof and other mixtures, such as enantiomer or diastereomer enriched mixtures, all of which fall within the scope of the present disclosure. Additional asymmetric carbon atoms may exist in alkyl and other substituents. All of these isomers and their mixtures are included within the scope of the present disclosure.

Optically-active (R)- and (S)- isomers, as well as D and L isomers, can be prepared through chiral synthesis or chiral reagents or other conventional techniques. If an enantiomer of a certain compound of the present disclosure wants to be obtained, the enantiomer can be prepared by asymmetric synthesis or by derivatization with a chiral auxiliary, wherein the resulting diastereomer mixture is separated, and an auxiliary group is cleaved to provide the pure desired enantiomer. Alternatively, when the molecules contain alkaline functional groups (such as amino) or acidic functional groups (such as carboxyl), a salt of the diastereomer is formed with an appropriate optically-active acid or alkali, then diastereomer separation is performed using conventional methods known in the art, and then recovery is performed to obtain the pure enantiomer. In addition, the separation of the enantiomer and the diastereomer is typically achieved using chromatography, and the chromatography employs a chiral stationary phase and is optionally combined with a chemical derivatization method (such as generating carbamate from amine).

"Optional" or "optionally" means that the subsequently described event or circumstance may possibly but not necessarily occur, and that the description includes an instance where the event or circumstance occurs and an instance where the event or circumstance does not occur.

The compound of the present disclosure may be prepared by various synthetic methods well-known to those skilled in the art, including the specific implementations listed below, the implementations formed by combination of the specific implementations with other chemical synthesis methods, and equivalent substitution manners well-known to those skilled in the art. Preferred implementations include but are not limited to the embodiments of the present disclosure.

Compared to the prior art, beneficial effects of the present disclosure include:
The compound of the present disclosure is the endothelin A (ETA) receptor antagonist compound, and this prodrug can be completely transformed into Atrasentan in human liver microsomes and has a technical effect comparable to Atrasentan in terms of pharmacokinetic study.

The salt of the compound represented by formula (I) provided by the present disclosure has improved physical and chemical properties compared to a free compound represented by (I), making it more suitable for pharmaceutical applications.

### BRIEF DESCRIPTION OF THE DRAWINGS

FIG. 1 is a schematic diagram of a structural formula of a salt of a compound A in the present disclosure.
FIG. 2 is a nuclear magnetic spectrum of hydrochloride obtained in Embodiment 18 of the present disclosure.
FIG. 3 is a nuclear magnetic spectrum of sulfate obtained in Embodiment 19 of the present disclosure.

### DETAILED DESCRIPTION

The present disclosure will be further described in detail below with reference to embodiments and accompanying drawings, but the implementations of the present disclosure are not limited to these.

A structure of a compound is determined by nuclear magnetic resonance (NMR) or mass spectrometry (MS). NMR shift (δ) is given in units of 10⁻⁶ (ppm). The determination of the NMR is carried out using a Bruker AVANCE-III NMR instrument, with deuterated dimethyl sulfoxide (DMSO-d₆) and deuterated chloroform (CDCl₃) as solvents, and tetramethylsilane (TMS) as the internal standard.

The determination of the MS is carried out using an ISQ EC mass spectrometer (manufacturer: Thermo, model: ISQ EC).

High performance liquid chromatography (HPLC) analysis is performed using a Thermo U3000 HPLC DAD high performance liquid chromatograph.

A CombiFlash rapid preparation instrument uses CombiFlash Rf+LUMEN (TELEDYNE ISCO).

A thin layer chromatography silica gel plate uses a HSGF₂₅₄ or GF₂₅₄ silica gel plate from Yantai Yinlong. The specification of the silica gel plate used for thin layer chromatography (TLC) is in a range from 0.17 mm to 0.23 mm, and the specification of a thin layer chromatography separation and purification product is in a range from 0.4 mm to 0.5 mm.

A silica gel column chromatography generally uses 100-200 meshes of Rushan Sanpont New silica gel as a carrier.

The present disclosure relates to the following reagents: N,N-dimethylformamide (DMF), potassium iodide (KI), cesium carbonate (Cs₂CO₃), dichloromethane (DCM), n-hexane, ethyl acetate (EA), pyridine (Py), trifluoroacetic acid (CF₃COOH), isopropanol (IPA), isopropyl ether, and acetone.

### Embodiment 1

### 1-[(ethoxycarbonyl)oxy]methyl-(2R,3R,4S)-4-(benzo[d][1,3]dioxolane-5-yl)-1-[2-(d ibutylamine)-2-oxoethyl]-2-(4-methoxyphenyl)pyrrolidine-3-carboxylate

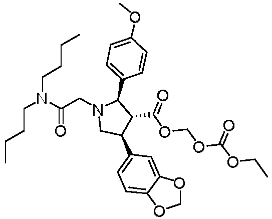

### Step A: 1-[(ethoxycarbonyl)oxy]methyl(2R,3R,4S)-4-(benzo[d][1,3]dioxolane-5-yl)-1-[2-(dibutyl amino)-2-oxoethyl]-2-(4-methoxyphenyl)pyrrolidine-3-carboxylate was synthesized.

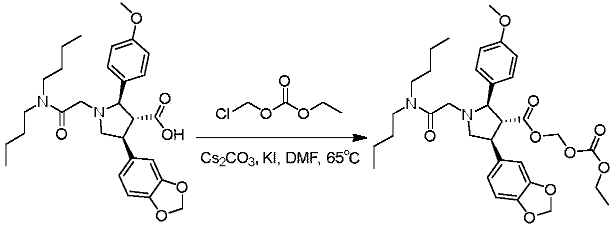

At room temperature, Atrasentan (2R,3R,4S)-4-(benzo [d][1,3]dioxolan-5-yl)-1-[2-(dibutylamino)-2-oxoethyl]-2-(4-methoxyphenyl)pyrrolidine-3-carboxylic acid (500 mg, 0.98 mmol), chloromethyl ethyl carbonate (270 mg, 1.96 mmol), cesium carbonate (640 mg, 1.96 mmol), and potassium iodide (325 mg, 1.96 mmol) were added to 10 mL of dry DMF and heated to 65°C to react for 2 hours.

After the reaction was completed, a reaction solution was cooled to room temperature, poured into 40 mL of an ice water solution, and extracted with dichloromethane (100 mL×3). Organic phases were combined. A mixture was washed with saturated salt water (50 mL), dried with anhydrous magnesium sulfate, filtered, concentrated under reduced pressure and dried by distillation. A crude compound was purified by silica-gel column chromatography (eluent: n-hexane/ethyl acetate=1/1) to obtain 480 mg of a colorless oily product [(ethoxycarbonyl)oxy]methyl(2R,3R,4S)-4-(benzo[d][1,3]dioxolane-5-yl)-1-[2-((dibutylami no)-2-oxoethyl]-2-(4-methoxyphenyl)pyrrolidine-3-carboxylate (yield: 79.9%).

LC-MS:RT = 2.20 min, [M+H]⁺ =613.42.

¹H NMR (400 MHz, DMSO) δ 7.23 (d, *J =* 8.6 Hz, 2H), 7.04 (d, *J =* 1.2 Hz, 1H), 6.89 (d, *J =* 8.7 Hz, 2H), 6.84-6.76 (m, 2H), 5.98 (d, *J =* 4.6 Hz, 2H), 5.58 (q, *J =* 6.2 Hz, 2H), 4.12 (q, *J =* 7.1 Hz, 2H), 3.78-3.72 (m, 1H), 3.72 (s, 3H), 3.49 (dd, *J =* 11.2, 5.3 Hz, 1H), 3.26-3.20 (m, 3H), 3.16 (s, 1H), 3.02-2.90 (m, 3H), 2.86-2.80 (m, 1H), 2.71 (d, *J =* 13.8 Hz, 1H), 1.33 (dd, *J =* 14.6, 7.0 Hz, 2H), 1.27-1.09 (m, 7H), 0.96 (dd, *J =* 14.7, 7.3 Hz, 2H), 0.81 (t, *J =* 7.3 Hz, 3H), 0.71 (t, *J =* 7.3 Hz, 3H).

### Embodiment 2 1-[(ethoxycarbonyl)oxy]ethyl(2R,3R,4S)-4-(benzo[d][1,3]dioxolane-5-yl)-1-[2-(dib utylamino)-2-oxoethyl]-2-(4-methoxyphenyl)pyrrolidine-3-carboxylate

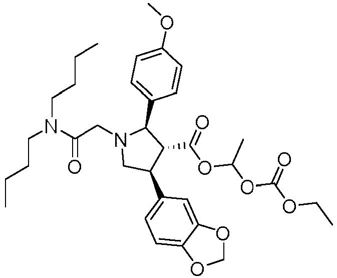

### Step A: 1-[(ethoxycarbonyl)oxy]ethyl(2R,3R,4S)-4-(benzo[d][1,3]dioxolane-5-yl)-1-[2-(dibutyla mino)-2-oxoethyl]-2-(4-methoxyphenyl)pyrrolidine-3-carboxylate was synthesized.

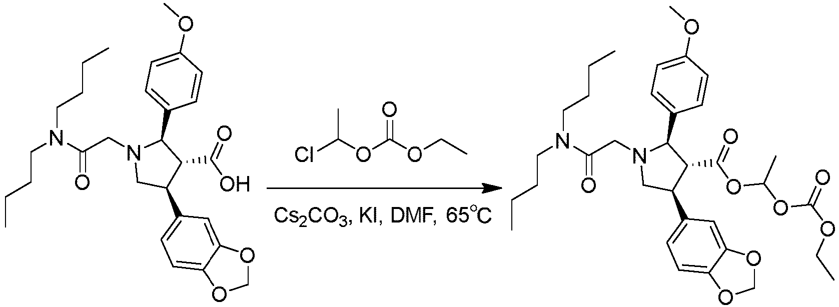

At room temperature, Atrasentan (2R,3R,4S)-4-(benzo [d][1,3]dioxolane-5-yl)-1-[2-(dibutylamino)-2-oxoethyl]-2-(4-methoxyphenyl)pyrrolidine-3-carboxylic acid (500 mg, 0.98 mmol), 1-chloroethyl ethyl carbonate (298 mg, 1.96 mmol), cesium carbonate (640 mg, 1.96 mmol), and potassium iodide (325 mg, 1.96 mmol) were added to 10 mL of dry DMF and heated to 65°C to react for 2 hours.

After the reaction was completed, a reaction solution was cooled to room temperature, poured into 40 mL of an ice water solution, and extracted with dichloromethane (100 mL×3). Organic phases were combined. A mixture was washed with saturated salt water (50 mL), dried with anhydrous magnesium sulfate, filtered, concentrated under reduced pressure and dried by distillation. A crude compound was purified by silica-gel column chromatography (eluent: n-hexane/ethyl acetate=1/1) to obtain 442 mg of a colorless oily product 1-[(ethoxycarbonyl)oxy]methyl(2R,3R,4S)-4-(benzo[d][1,3]dioxolane-5-yl)-1-[2-(dibutylam ino)-2-oxoethyl]-2-(4-methoxyphenyl)pyrrolidine-3-carboxylate (yield: 66.5%).

LC-MS:RT = 2.21 min, [M+H]⁺ =627.40. ¹H NMR (400 MHz, DMSO) δ 7.27-7.17 (m, 2H), 7.09-6.98 (m, 1H), 6.92-6.85 (m, 2H), 6.85-6.74 (m, 2H), 6.60-6.52 (m, 1H), 5.98 (d, *J* = 5.3 Hz, 2H), 4.11 (dq, *J* = 11.1, 7.1 Hz, 2H), 3.67-3.73 (m, 4H), 3.57-3.44 (m, 1H), 3.30-3.20 (m, 3H), 3.19-3.10 (m, 1H), 3.01-2.85 (m, 3H), 2.77 (ddd, *J =* 9.4, 6.9, 2.6 Hz, 1H), 2.70 (d, *J =* 13.9 Hz, 1H), 1.39-1.22 (m, 6H), 1.21-1.04 (m, 6H), 1.01-0.89 (m, 2H), 0.81 (t, *J =* 7.3 Hz, 3H), 0.71 (t, *J =* 7.3 Hz, 3H)

### Embodiment 3 1-[(isopropoxycarbonyl)oxy]ethyl-(2R,3R,4S)-4-(benzo[d][1,3]dioxolane-5-yl)-1-[2-(dibutylamino)-2-oxoethyl]-2-(4-methoxyphenyl)pyrrolidine-3-carboxylate

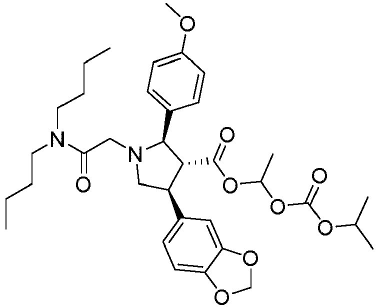

### Step A: 1- [(isopropoxycarbonyl)oxy] ethyl-(2R,3R,4S)-4-(benzo[d][1,3]dioxolane-5-yl)-1-[2-(dib utylamino)-2-oxoethyl]-2-(4-methoxyphenyl)pyrrolidine-3-carboxylate was synthesized.

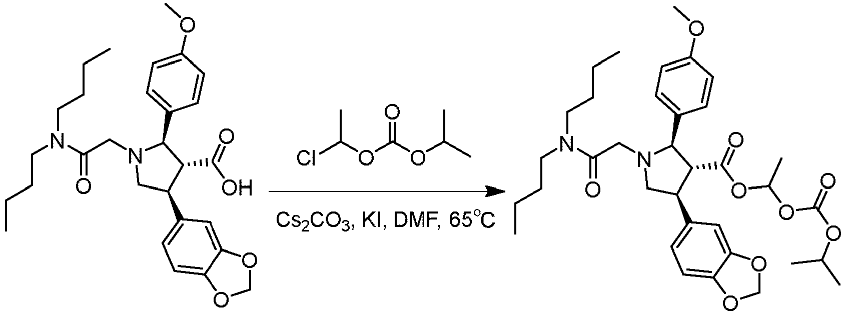

At room temperature, Atrasentan (2*R*,3*R*,4*S*)-4-(benzo[*d*][1,3]dioxolane-5-yl)-1-[2-(dibutylamino)-2-oxoethyl]-2-(4-methoxy phenyl)pyrrolidine-3-carboxylic acid (500 mg, 0.98 mmol), 1-chloroethyl isopropyl carbonate (325 mg, 1.96 mmol), cesium carbonate (640 mg, 1.96 mmol), and potassium iodide (325 mg, 1.96 mmol) were added to 10 mL of dry DMF and heated to 65°C to react for 2 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=1/1) to obtain 482 mg of a colorless oily product **1-[(isopropoxycarbonyl)oxy]ethyl-(2*R*,3*R*,4*S*)-4-(benzo[*d*][1,3]dioxolane-5-yl)-1-[2-(dibutyl** amino)-2-oxoethyl]-2-(4-methoxyphenyl)pyrrolidine-3-carboxylate (yield: 76.9%).

LC-MS:RT = 2.29 min, [M+H]⁺ =641.48. ¹H NMR (400 MHz, DMSO) δ 7.25 (t, *J* = 9.0 Hz, 2H), 7.07-7.04 (m, 1H), 6.93-6.89 (m, 2H), 6.85-6.78 (m, 2H), 6.59-6.54 (m, 1H), 5.99 (d, *J =* 6.2 Hz, 2H), 4.79-4.69 (m, 1H), 3.76-3.70 (m, 4H), 3.55-3.44 (m, 1H), 3.33-3.23 (m, 4H), 3.21-3.13 (m, 1H), 3.02-2.90 (m, 3H), 2.80-2.75 (m, 1H)2.71 (d, *J =* 13.9 Hz, 1H), 1.39-1.33 (m, 1H), 1.32 (d, *J =* 5.4 Hz, 2H), 1.26 (d, *J =* 5.4 Hz, 2H), 1.22-1.14 (m, 9H), 1.00-0.94 (m, 2H), 0.82 (t, *J =* 7.3 Hz, 3H), 0.72 (t, *J =* 7.3 Hz, 3H).

### Embodiment 4 1-[(methoxycarbonyl)oxy]ethyl-(2R,3R,4S)-4-(benzo[d][1,3]dioxolane-5-yl)-1-[2-(di butylamino)-2-oxoethyl]-2-(4-methoxyphenyl)pyrrolidine-3-carboxylate

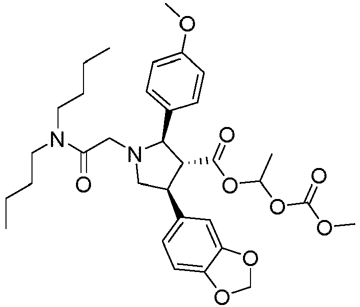

### Step A: 1-[(methoxycarbonyl)oxy]ethyl-(2R,3R,4S)-4-(benzo[d][1,3]dioxolane-5-yl)-1-[2-(dibuty lamino)-2-oxoethyl]-2-(4-methoxyphenyl)pyrrolidine-3-carboxylate was synthesized.

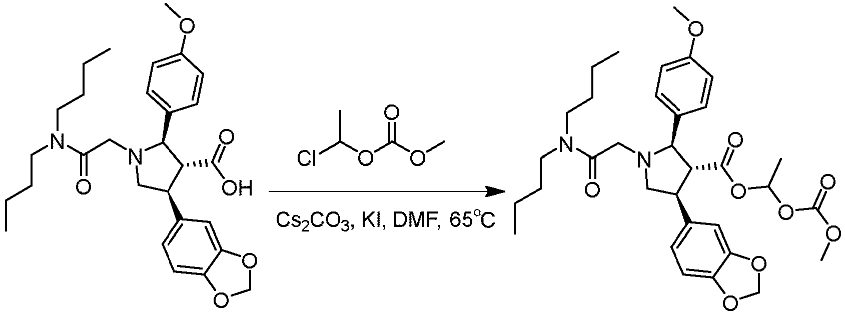

At room temperature, Atrasentan (2*R*,3*R*,4*S*)-4-(benzo[*d*][1,3]dioxolane-5-yl)-1-[2-(dibutylamino)-2-oxoethyl]-2-(4-methoxy phenyl)pyrrolidine-3-carboxylic acid (500 mg, 0.98 mmol), 1-chloroethyl methyl carbonate (270 mg, 1.96 mmol), cesium carbonate (640 mg, 1.96 mmol), and potassium iodide (325 mg, 1.96 mmol) were added to 10 mL of dry DMF and heated to 65°C to react for 2 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=1/1) to obtain 428 mg of a colorless oily product 1-[(methoxycarbonyl)oxy]ethyl-(2*R*,3*R*,4*S*)-4-(benzo[*d*][1,3]dioxolane-5-yl)-1-[2-(dibutyla mino)-2-oxoethyl]-2-(4-methoxyphenyl)pyrrolidine-3-carboxylate (yield: 71.3%).

LC-MS:RT = 2.20 min, [M+H]⁺ =613.42. ¹H NMR (400 MHz, DMSO) δ 7.25 (dd, *J* = 10.3, 8.7 Hz, 2H), 7.08-7.04 (m, 1H), 6.91 (dd, *J* = 8.7, 2.9 Hz, 2H), 6.86-6.78 (m, 2H), 6.60-6.55 (m, 1H), 5.99 (d, *J =* 5.1 Hz, 2H), 3.76-3.69 (m, 6H), 3.56-3.44 (m, 1H), 3.33-3.16 (m, 5H), 3.00-2.94 (m, 3H), 2.81-2.76 (m, 1H), 2.71 (d, *J =* 13.7 Hz, 1H), 1.39-1.14 (m, 9H), 1.02-0.93 (m, 2H), 0.82 (t, *J =* 7.3 Hz, 3H), 0.72 (t, *J =* 7.3 Hz, 3H).

### Embodiment 5 1-(((2-(methylamino)ethoxy)carbonyl)oxy)ethyl-(2R,3R,4S)-4-(benzo[d][1,3]dioxol ane-5-yl)-1-[2-(dibutylamino)-2-oxoethyl]-2-(4-methoxyphenyl)pyrrolidine-3-carboxyla te

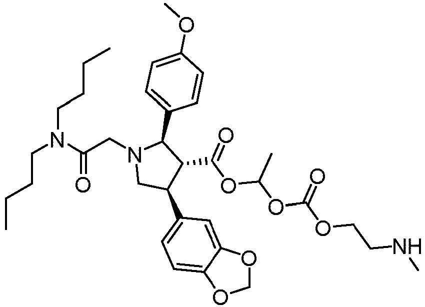

### Step A: (2-(((1-chloroethoxy)carbonyl)oxy)ethyl)(methyl)tert-butyl carbamate was synthesized.

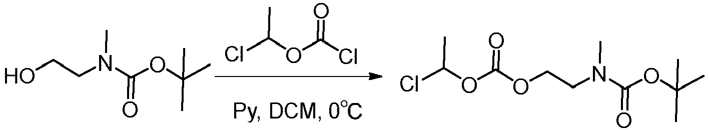

Under an ice bath, 2-(N-Boc-N-methylamino)ethanol (500 mg, 2.85 mmol) and pyridine (248 mg, 3.14 mmol) were added to 10 mL of dry dichloromethane. 1-chloroethyl chloroformate (248 mg, 3.14 mmol) was added dropwise under an ice bath, and after dripping, a mixture was heated to room temperature to react for 1 hour.

After the reaction was completed, a mixture was poured into 40 mL of an ice water solution, and extracted with dichloromethane (20 mL×3), and an organic layer was washed twice with 1 M/L hydrochloric acid (20 mL), washed with saturated salt solution (50 mL), dried with anhydrous magnesium sulfate, filtered, and concentrated under reduced pressure to dryness to obtain 725 mg of a colorless oily product (2-(((1-chloroethoxy)carbonyl)oxy)ethyl)(methyl)tert-butyl carbamate (yield: 90.6%).

### Step B: 2,2,5-trimethyl-4,9-dioxo-3,8,10-trioxy-5-aza-11-ethyl-(2R,3R,4S)-4-(benzo[d][1,3] dioxo lane-5-yl)-1-[2-(dibutylamino)-2-oxoethyl]-2-(4-methoxyphenyl)pyrrolidine-3-carboxyl ate was synthesized.

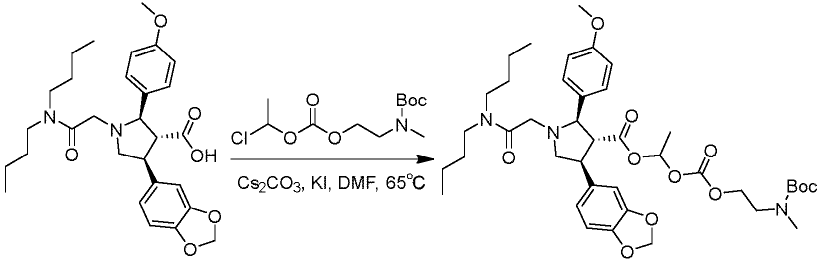

At room temperature, Atrasentan (2*R*,3*R*,4*S*)-4-(benzo[*d*][1,3]dioxolane-5-yl)-1-[2-(dibutylamino)-2-oxoethyl]-2-(4-methoxy phenyl)pyrrolidine-3-carboxylic acid (500 mg, 0.98 mmol), (2-(((1-chloroethoxy)carbonyl)oxy)ethyl)(methyl)tert-butyl carbamate (550 mg, 1.96 mmol), cesium carbonate (640 mg, 1.96 mmol), and potassium iodide (325 mg, 1.96 mmol) were added to 10 mL of dry DMF and heated to 65°C to react for 4 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=1/1) to obtain 625 mg of a colorless oily product 2,2,5-trimethyl-4,9-dioxo-3,8,10-trioxy-5-aza-11-ethyl-(2*R*,3*R*,4*S*)-4-(benzo[*d*][1,3]dioxolan e-5-yl)-1-[2-(dibutylamino)-2-oxoethyl]-2-(4-methoxyphenyl)pyrrolidine-3-carboxylate (yield: 84.4%). LC-MS:RT = 2.34 min, [M+H]⁺ =756.49.

### Step C: 1-(((2-(methylamino)ethoxy)carbonyl)oxy)ethyl-(2R,3R,4S)-4-(benzo[d][1,3]dioxolane-5 -yl)-1-[2-(dibutylamino)-2-oxoethyl]-2-(4-methoxyphenyl)pyrrolidine-3-carboxylate was synthesized.

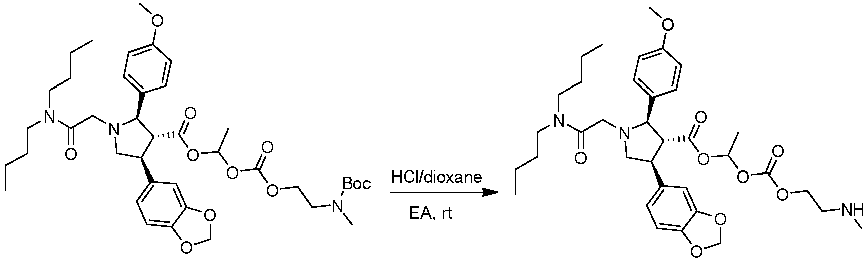

At room temperature, 2,2,5-trimethyl-4,9-dioxo-3,8,10-trioxy-5-aza-11-ethyl-(2*R*,3*R*,4*S*)-4-(benzo[*d*][1,3]dioxolan e-5-yl)-1-[2-(dibutylamino)-2-oxoethyl]-2-(4-methoxyphenyl)pyrrolidine-3-carboxylate (300 mg, 0.39 mmol) was dissolved in 10 mL of an ethyl acetate solvent, 2 mL of a 4M/L hydrochloric acid dioxane solution was added under an ice bath, and a reaction was carried out at room temperature for 2 hours.

After the reaction was completed, 260 mg of yellow solid 1-(((2-(methylamino)ethoxy)carbonyl)oxy)ethyl-(2*R*,3*R*,4*S*)-4-(benzo[*d*][1,3]dioxolane-5-yl )-1-[2-(dibutylamino)-2-oxoethyl]-2-(4-methoxyphenyl)pyrrolidine-3-carboxylate was obtained by concentration (yield: 101.6%).

LC-MS:RT = 1.82 min, [M+H]⁺ =656.45. ¹H NMR (400 MHz, DMSO) δ 7.60 (m, 2H), 7.21 (m, 1H), 7.01 (d, *J =* 8.1 Hz, 2H), 6.90 (d, *J =* 15.8 Hz, 2H), 6.50 (m, 1H), 6.04 (s, 2H), 3.77 (s, 3H), 3.17 (d, *J =* 6.4 Hz, 3H), 3.08-2.90 (m, 4H), 2.52 (d, *J =* 5.6 Hz, 3H), 2.06 (d, *J* = 4.1 Hz, 3H), 1.90 (s, 6H), 1.33 (d, *J =* 34.4 Hz, 4H), 1.23-1.10 (m, 7H), 0.83 (t, *J =* 7.0 Hz, 6H).

### Embodiment 6 1-((((S)-2,3-dihydroxypropoxy)carbonyl)oxy)ethyl-(2R,3R,4S)-4-(benzo[d][1,3]diox olane-5-yl)-1-[2-(dibutylamino)-2-oxoethyl]-2-(4-methoxyphenyl)pyrrolidine-3-carboxy late

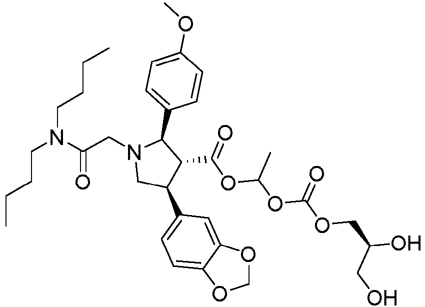

### step A: 1-chloroethyl(((S)-2,2-dimethyl-1,3-dioxolane-4-yl)methyl)carbonate

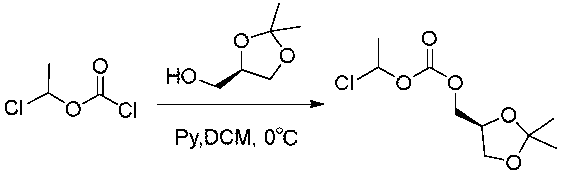

Under an ice bath, (S)-(+)-1,2-isopropylideneglycerol (315 mg, 2.38 mmol) and pyridine (225 mg, 2.85 mmol) were added to 10 mL of dry dichloromethane. 1-chloroethyl chloroformate (408 mg, 2.85 mmol) was added dropwise under an ice bath, and after dripping, a mixture was heated to room temperature to react for 2 hours.

After the reaction was completed, the mixture was poured into 40 mL of an ice water solution, extracted with dichloromethane (20 mL×3), washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=10/1) to obtain 250 mg of a colorless oily product 1-chloroethyl(((S)-2,2-dimethyl-1,3-dioxolane-4-yl)methyl)carbonate (yield: 44.2%).

### Step B: 1-(((((S)-2,2-dimethyl-1,3-dioxolane-4-yl)methoxy)carbonyl)oxy)ethyl-(2R,3R,4S)-4-(be nzo[d][1,3]dioxolane-5-yl)-1-[2-(dibutylamino)-2-oxoethyl]-2-(4-methoxyphenyl)pyrroli dine-3-carboxylate was synthesized.

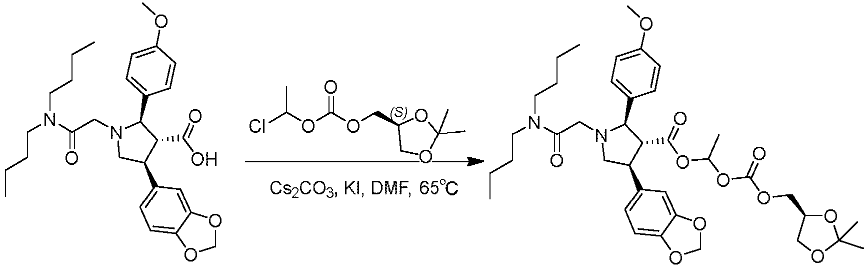

At room temperature, Atrasentan (2*R*,3*R*,4*S*)-4-(benzo[*d*][1,3]dioxolane-5-yl)-1-[2-(dibutylamino)-2-oxoethyl]-2-(4-methoxy phenyl)pyrrolidine-3-carboxylic acid (100 mg, 0.20 mmol), 1-chloroethyl(((S)-2,2-dimethyl-1,3-dioxolane-4-yl)methyl)carbonate (72 mg, 0.3 mmol), cesium carbonate (130 mg, 0.4 mmol), and potassium iodide (66.4 mg, 0.4 mmol) were added to 5 mL of dry DMF and heated to 65°C to react for 4 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=3/1) to obtain 85 mg of a colorless oily product 1-(((((S)-2,2-dimethyl-1,3-dioxolane-4-yl)methoxy)carbonyl)oxy)ethyl-(2*R*,3*R*,4*S*)-4-(benzo [*d*][1,3]dioxolane-5-yl)-1-[2-(dibutylamino)-2-oxoethyl]-2-(4-methoxyphenyl)pyrrolidine-3-carboxylate (yield: 59.9%). LC-MS:RT = 2.21 min, [M+H]⁺ =713.46.

### Step C: 1-((((S)-2,3-dihydroxypropoxy)carbonyl)oxy)ethyl-(2R,3R,4S)-4-(benzo[d][1,3]dioxolan e-5-yl)-1-[2-(dibutylamino)-2-oxoethyl]-2-(4-methoxyphenyl)pyrrolidine-3-carboxylate was synthesized.

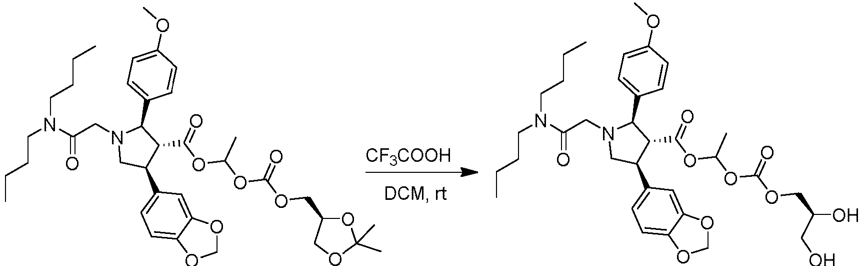

At room temperature, 1-(((((S)-2,2-dimethyl-1,3-dioxolane-4-yl)methoxy)carbonyl)oxy)ethyl-(2R,3R,4S)-4-(benz o[*d*][1,3]dioxolane-5-yl)-1-[2-(dibutylamino)-2-oxoethyl]-2-(4-methoxyphenyl)pyrrolidine-3-carboxylate (80 mg, 0.11 mmol) was dissolved in 10 mL of a dichloromethane solvent, 2 mL of a trifluoroacetic acid solution was added, and a reaction was carried out at room temperature for 0.5 hour.

After the reaction was completed, a crude compound was purified by silica-gel column chromatography (eluent: n-hexane/ethyl acetate=1/2) to obtain 56 mg of yellow solid 1-((((S)-2,3-dihydroxypropoxy)carbonyl)oxy)ethyl-(2*R*,3*R*,4*S*)-4-(benzo[*d*][1,3]dioxolane-5 -yl)-1-[2-(dibutylamino)-2-oxoethyl]-2-(4-methoxyphenyl)pyrrolidine-3-carboxylate (yield: 74.1%).

LC-MS:RT = 2.06 min, [M+H]⁺ =673.39. ¹H NMR (400 MHz, DMSO) δ 7.32-7.17 (m, 2H), 7.12-7.04 (m, 1H), 6.93 (s, 2H), 6.84 (s, 2H), 6.55 (s, 1H), 6.00 (d, *J =* 5.2 Hz, 2H), 4.18-4.06 (m, 2H), 3.90 (s, 1H), 3.73 (s, 3H), 3.64 (s, 2H), 3.55-3.42 (m, 2H), 3.18-3.07 (m, 3H), 3.00 (s, 4H), 2.66 (s, 1H), 2.44-2.35 (m, 2H), 2.32 (s, 1H), 1.31 (s, 3H), 1.18 (s, 3H), 0.99 (s, 3H), 0.81 (t, *J =* 7.4 Hz, 3H), 0.74 (s, 3H).

### Embodiment 7 1-[(cyclopropoxycarbonyl)oxy]ethyl-(2R,3R,4S)-4-(benzo[d][1,3]dioxolane-5-yl)-1-[ 2-(dibutylamino)-2-oxoethyl]-2-(4-methoxyphenyl)pyrrolidine-3-carboxylate

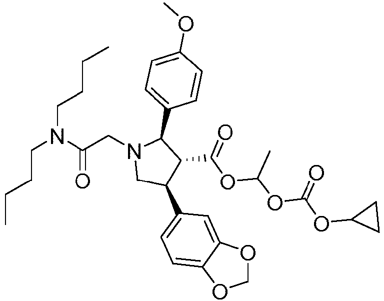

### Step A: 1-[(cyclopropoxycarbonyl)oxy]ethyl-(2R,3R,4S)-4-(benzo[d][1,3]dioxolane-5-yl)-1-[2-(di butylamino)-2-oxoethyl]-2-(4-methoxyphenyl)pyrrolidine-3-carboxylate was synthesized.

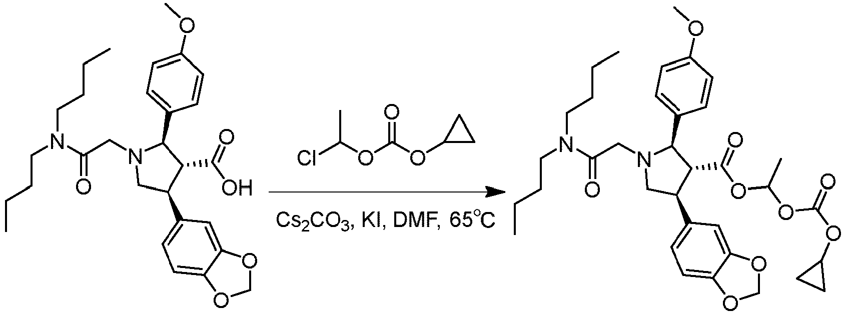

At room temperature, Atrasentan (2*R*,3*R*,4*S*)-4-(benzo[*d*][1,3]dioxolane-5-yl)-1-[2-(dibutylamino)-2-oxoethyl]-2-(4-methoxyphenyl)pyrrolidine-3-carboxylic acid (500 mg, 0.98 mmol), 1-chloroethyl cyclopropyl carbonate (240 mg, 1.96 mmol), cesium carbonate (640 mg, 1.96 mmol), and potassium iodide (325 mg, 1.96 mmol) were added to 10 mL of dry DMF and heated to 65°C to react for 2 hours.

After the reaction was completed, a mixture was cooled to room temperature, poured into 40 mL of an ice water solution, and extracted with dichloromethane (100 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=1/1) to obtain 412 mg of a colorless oily product 1-[(cyclopropoxycarbonyl)oxy]ethyl-(2*R*,3*R*,4*S*)-4-(benzo[*d*][1,3]dioxolane-5-yl)-1-[2-(dibu tylamino)-2-oxoethyl]-2-(4-methoxyphenyl)pyrrolidine-3-carboxylate (yield: 65.8%). LC-MS:RT = 2.29 min, [M+H]⁺ =639.40. ¹H NMR (400 MHz, DMSO) δ 7.25 (s, 2H), 7.06 (d, *J =* 10.4 Hz, 1H), 6.90 (m, 2H), 6.83 (m, 2H), 6.57 (m, 1H), 5.98 (s, 2H), 4.09 (s, 2H), 3.73 (m, 4H), 3.53-3.42 (m, 2H), 2.95 (m, 5H), 2.71 (d, *J* = 13.2 Hz, 3H), 1.98 (s, 1H), 1.38-1.02 (m, 9H), 0.99-0.87 (m, 2H), 0.81 (s, 2H), 0.71 (d, *J =* 7.0 Hz, 6H).

### Embodiment 8 1-(((2-hydroxyethoxy)carbonyl)oxy)ethyl-(2R,3R,4S)-4-(benzo[d][1,3]dioxolane-5-yl)-1-[2-(dibutylamino)-2-oxoethyl]-2-(4-methoxyphenyl)pyrrolidine-3-carboxylate

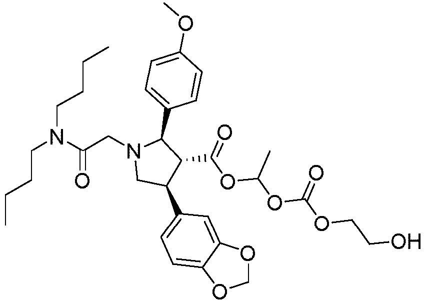

### Step A: 2-((tert-butyldimethylsilyl)oxy)ethyl(1-chloroethyl)carbonate was synthesized.

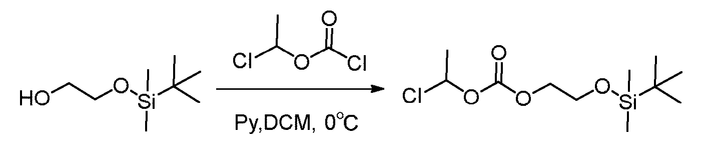

Under an ice bath, 2-tert-butyldimethylsiloxyethanol (1000 mg, 5.67 mmol) and pyridine (739 mg, 9.36 mmol) were added to 10 mL of dry dichloromethane. 1-chloroethyl chloroformate (900 mg, 6.24 mmol) was added dropwise under an ice bath, and after dripping, a mixture was heated to room temperature to react for 1 hour.

After the reaction was completed, the mixture was poured into 40 mL of an ice water solution, extracted with dichloromethane (20 mL×3), washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=10/1) to obtain 245 mg of a colorless oily product 2-((tert-butyldimethylsilyl)oxo)ethyl(1-chloroethyl)carbonate (yield: 15.4%).

### Step B: 2,2,3,3-tetramethyl-8-oxo-4,7,9-trioxa-3-sila-10-ethyl-(2R,3R,4S)-4-(benzo[d] [1,3]dioxol ane-5-yl)-1-[2-(dibutylamino)-2-oxoethyl]-2-(4-methoxyphenyl)pyrrolidine-3-carboxyla te was synthesized.

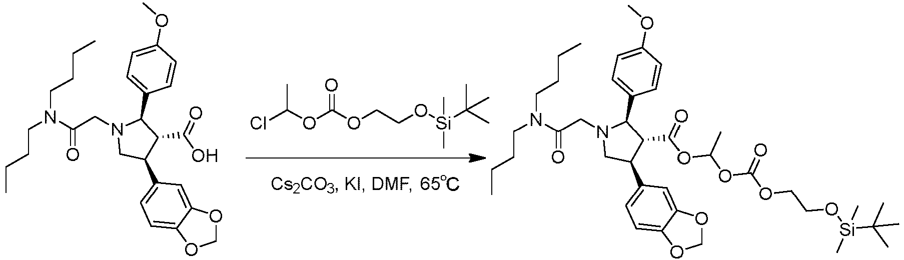

At room temperature, Atrasentan (2*R*,3*R*,4*S*)-4-(benzo[*d*][1,3]dioxolane-5-yl)-1-[2-(dibutylamino)-2-oxoethyl]-2-(4-methoxy phenyl)pyrrolidine-3-carboxylic acid (100 mg, 0.20 mmol), 2-((tert-butyldimethylsilyl)oxy)ethyl(1-chloroethyl)carbonate (85 mg, 0.3 mmol), cesium carbonate (130 mg, 0.4 mmol), and potassium iodide (66.4 mg, 0.4 mmol) were added to 5 mL of dry DMF and heated to 65°C to react for 4 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=3/1) to obtain 62 mg of a colorless oily product 2,2,3,3-tetramethyl-8-oxo-4,7,9-trioxa-3-sila-10-ethyl-(2*R*,3*R*,4*S*)-4-(benzo[*d*][1,3]dioxolane -5-yl)-1-[2-(dibutylamino)-2-oxoethyl]-2-(4-methoxyphenyl)pyrrolidine-3-carboxylate (yield: 41.0%). LC-MS:RT = 2.75 min, [M+H]⁺ =757.50.

### Step C: 1-(((2-(hydroxyethoxy)carbonyl)oxy)ethyl-(2R,3R,4S)-4-(benzo[d][1,3]dioxolane-5-yl)-1 -[2-(dibutylamino)-2-oxoethyl]-2-(4-methoxyphenyl)pyrrolidine-3-carboxylate was synthesized.

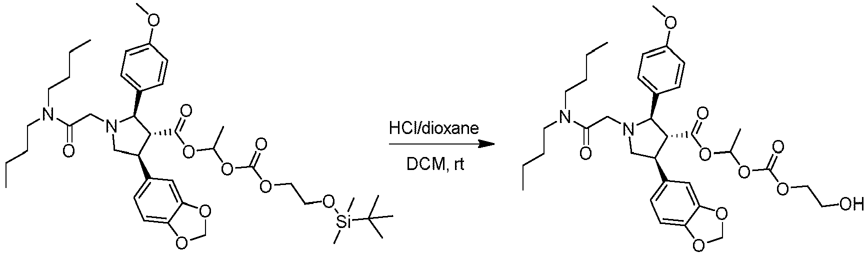

At room temperature, 2,2,3,3-tetramethyl-8-oxo-4,7,9-trioxa-3-sila-10-ethyl-(2*R*,3*R*,4*S*)-4-(benzo[*d*][1,3]dioxolane -5-yl)-1-[2-(dibutylamino)-2-oxoethyl]-2-(4-methoxyphenyl)pyrrolidine-3-carboxylate (62 mg, 0.08 mmol) was dissolved in 10 mL of a dichloromethane solvent, 2 mL of a 4M/L hydrochloric acid dioxane solution was added under an ice bath, and a reaction was carried out at room temperature for 2 hours.

After the reaction was completed, a crude compound was purified by silica-gel column chromatography (eluent: n-hexane/ethyl acetate=1/2) to obtain 40 mg of yellow solid 1-(((2-hydroxyethoxy)carbonyl)oxy)ethyl-(2*R*,3*R*,4*S*)-4-(benzo[*d*][1,3]dioxolane-5-yl)-1-[2-(dibutylamino)-2-oxoethyl]-2-(4-methoxyphenyl)pyrrolidine-3-carboxylate (yield: 77.9%).

LC-MS:RT = 2.07 min, [M+H]⁺ =643.44. ¹H NMR (400 MHz, DMSO) δ 7.71-7.38 (m, 2H), 7.19 (d, *J =* 12.2 Hz, 1H), 7.00 (d, *J =* 6.8 Hz, 2H), 6.93-6.79 (m, 2H), 6.48 (s, 1H), 6.03 (s, 2H), 4.02 (d, *J =* 7.1 Hz, 3H), 3.76 (m, 4H), 3.57-3.48 (m, 3H), 3.20-3.09 (m, 1H), 3.08-2.90 (m, 3H), 1.45-1.02 (m, 14H), 0.91-0.68 (m, 8H).

### Embodiment 9 1-(((((R)-1,4-dioxane-2-yl)methoxy)carbonyl)oxy)ethyl-(2R,3R,4S)-4-(benzo[d][1,3] dioxolane-5-yl)-1-[2-(dibutylamino)-2-oxoethyl]-2-(4-methoxyphenyl)pyrrolidine-3-car boxylate

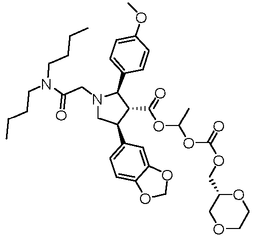

### Step A: ((R)-1,4-dioxane-2-yl)methyl(1-chloroethyl)carbonate

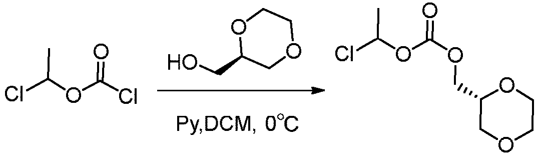

Under an ice bath, (*R*)-(1,4-dioxane-2-yl)methanol (280 mg, 2.38 mmol) and pyridine (225 mg, 2.85 mmol) were added to 10 mL of dry dichloromethane. 1-chloroethyl chloroformate (408 mg, 2.85 mmol) was added dropwise under an ice bath, and after dripping, a mixture was heated to room temperature to react for 2 hours.

After the reaction was completed, the mixture was poured into 40 mL of an ice water solution, extracted with dichloromethane (20 mL×3), washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=10/1) to obtain 212 mg of a colorless oily product ((*R*)-1,4-dioxane-2-yl)methyl(1-chloroethyl)carbonate (yield: 39.8%).

### Step B: 1-(((((R)-1,4-dioxane-2-yl)methoxy)carbonyl)oxy)ethyl-(2R,3R,4S)-4-(benzo[d][1,3]diox olane-5-yl)-1-[2-(dibutylamino)-2-oxoethyl]-2-(4-methoxyphenyl)pyrrolidine-3-carboxy late was synthesized.

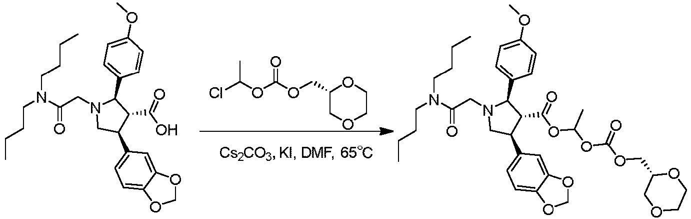

At room temperature, Atrasentan (2*R*,3*R*,4*S*)-4-(benzo[*d*][1,3]dioxolane-5-yl)-1-[2-(dibutylamino)-2-oxoethyl]-2-(4-methoxy phenyl)pyrrolidine-3-carboxylic acid (100 mg, 0.20 mmol), ((*R*)-1,4-dioxane-2-yl)methyl(1-chloroethyl)carbonate (67.2 mg, 0.3 mmol), cesium carbonate (130 mg, 0.4 mmol), and potassium iodide (66.4 mg, 0.4 mmol) were added to 5 mL of dry DMF and heated to 65°C to react for 4 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=3/1) to obtain 80 mg of a colorless oily product 1-(((((*R*)-1,4-dioxane-2-yl)methoxy)carbonyl)oxy)ethyl-(2*R*,3*R*,4*S*)-4-(benzo[*d*][1,3]dioxola ne-5-yl)-1-[2-(dibutylamino)-2-oxoethyl]-2-(4-methoxyphenyl)pyrrolidine-3-carboxylate (yield: 59.9%).

LC-MS:RT = 2.16 min, [M+H]⁺ =699.42. ¹H NMR (400 MHz, DMSO) δ 7.24 (dd, *J* = 11.6, 8.6 Hz, 2H), 7.06 (d, *J =* 11.4 Hz, 1H), 6.91 (dd, *J =* 8.6, 2.7 Hz, 2H), 6.85-6.75 (m, 2H), 6.59-6.53 (m, 1H), 5.99 (d, *J =* 6.0 Hz, 2H), 4.06 (dd, *J =* 9.5, 4.7 Hz, 2H), 3.75-3.56 (m, 8H), 3.53 (d, *J =* 9.9 Hz, 1H), 3.50-3.41 (m, 2H), 3.29-3.21 (m, 5H), 2.95 (dd, *J =* 18.3, 10.6 Hz, 3H), 2.79-2.73 (m, 1H), 2.69 (d, *J* = 14.1 Hz, 1H), 1.33 (t, *J* = 8.5 Hz, 3H), 1.29-1.21 (m, 3H), 1.17 (dd, *J =* 14.8, 7.4 Hz, 3H), 0.96 (d, *J = 4.7* Hz, 2H), 0.81 (t, *J =* 7.3 Hz, 3H), 0.71 (t, *J =* 7.3 Hz, 3H).

### Embodiment 10 1-((((oxetane-3-yl)oxy)carbonyl)oxy)ethyl-(2R,3R,4S)-4-(benzo[d][1,3]dioxolane-5-yl)-1-[2-(dibutylamino)-2-oxoethyl]-2-(4-methoxyphenyl)pyrrolidine-3-carboxylate

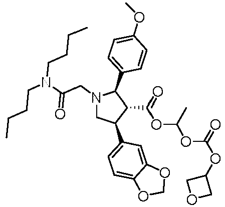

### Step A: 1-chloroethyloxetane-3-yl carbonate

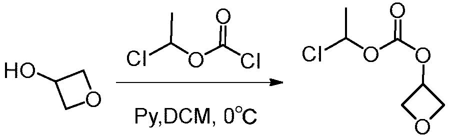

Under an ice bath, oxetane-3-ol (176 mg, 2.38 mmol) and pyridine (225 mg, 2.85 mmol) were added to 10 mL of dry dichloromethane. 1-chloroethyl chloroformate (408 mg, 2.85 mmol) was added dropwise under an ice bath, and after dripping, a mixture was heated to room temperature to react for 2 hours.

After the reaction was completed, the mixture was poured into 40 mL of an ice water solution, extracted with dichloromethane (20 mL×3), washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=10/1) to obtain 200 mg of a colorless oily product 1-chloroethyloxetane-3-yl carbonate (yield: 46.7%).

### Step B: 1-((((oxetane-3-yl)oxy)carbonyl)oxy)ethyl-(2R,3R,4S)-4-(benzo[d][1,3]dioxolane-5-yl)-1-[2-(dibutylamino)-2-oxoethyl]-2-(4-methoxyphenyl)pyrrolidine-3-carboxylate was synthesized.

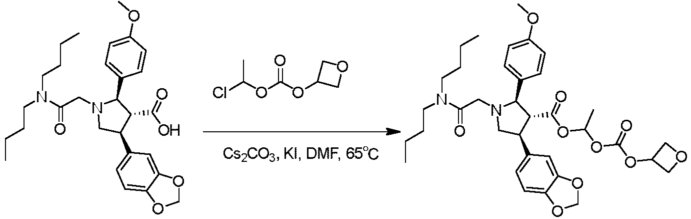

At room temperature, Atrasentan (2*R*,3*R*,4*S*)-4-(benzo[*d*][1,3]dioxolane-5-yl)-1-[2-(dibutylamino)-2-oxoethyl]-2-(4-methoxyphenyl)pyrrolidine-3-carboxylic acid (100 mg, 0.2 mmol), 1-chloroethyloxetane-3-yl carbonate (54 mg, 0.3 mmol), cesium carbonate (130 mg, 0.4 mmol), and potassium iodide (66.4 mg, 0.4 mmol) were added to 5 mL of dry DMF and heated to 65°C to react for 4 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=3/1) to obtain 76 mg of a colorless oily product 1-((((oxetane-3-yl)oxy)carbonyl)oxy)ethyl-(2*R*,3*R*,4*S*)-4-(benzo[*d*][1,3]dioxolane-5-yl)-1-[2 -(dibutylamino)-2-oxoethyl]-2-(4-methoxyphenyl)pyrrolidine-3-carboxylate (yield: 58.0%).

LC-MS:RT = 2.18 min, [M+H]⁺ =655.39. ¹H NMR (400 MHz, DMSO) δ 7.24 (t, *J =* 8.4 Hz, 2H), 7.05 (d, *J =* 12.1 Hz, 1H), 6.94-6.86 (m, 2H), 6.85-6.71 (m, 2H), 6.61-6.50 (m, 1H), 5.99 (d, *J* = 5.7 Hz, 2H), 5.41-5.26 (m, 1H), 4.82-4.63 (m, 2H), 4.53-4.32 (m, 2H), 3.80-3.60 (m, 4H), 3.57-3.41 (m, 1H), 3.27-3.11 (m, 4H), 2.98 (dd, *J =* 14.1, 6.6 Hz, 3H), 2.82-2.74 (m, 1H), 2.68 (s, 1H), 1.32 (t, *J =* 7.1 Hz, 3H), 1.26 (dd, *J =* 18.2, 7.3 Hz, 3H), 1.17 (dd, *J =* 14.8, 7.1 Hz, 3H), 0.96 (d, *J =* 6.6 Hz, 2H), 0.81 (t, *J =* 7.3 Hz, 3H), 0.71 (t, *J =* 7.3 Hz, 3H).

### Embodiment 11 1-(((((S)-1,4-dioxane-2-yl)methoxy)carbonyl)oxy)ethyl-(2R,3R,4S)-4-(benzo[d][1,3] dioxolane-5-yl)-1-[2-(dibutylamino)-2-oxoethyl]-2-(4-methoxyphenyl)pyrrolidine-3-car boxylate

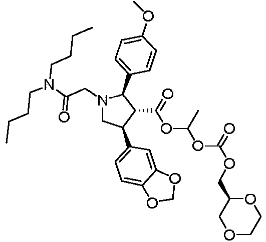

### Step A: ((S)-1,4-dioxane-2-yl)methyl(1-chloroethyl)carbonate

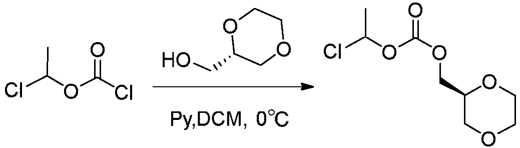

Under an ice bath, (*S*)-(1,4-dioxane-2-yl)methanol (280 mg, 2.38 mmol) and pyridine (225 mg, 2.85 mmol) were added to 10 mL of dry dichloromethane. 1-chloroethyl chloroformate (408 mg, 2.85 mmol) was added dropwise under an ice bath, and after dripping, a mixture was heated to room temperature to react for 2 hours.

After the reaction was completed, the mixture was poured into 40 mL of an ice water solution, extracted with dichloromethane (20 mL×3), washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=10/1) to obtain 219 mg of a colorless oily product ((*S*)-1,4-dioxane-2-yl)methyl(1-chloroethyl)carbonate (yield: 41.0%).

### Step B: 1-(((((S)-1,4-dioxane-2-yl)methoxy)carbonyl)oxy)ethyl-(2R,3R,4S)-4-(benzo[d][1,3]diox olane-5-yl)-1-[2-(dibutylamino)-2-oxoethyl]-2-(4-methoxyphenyl)pyrrolidine-3-carboxy late was synthesized.

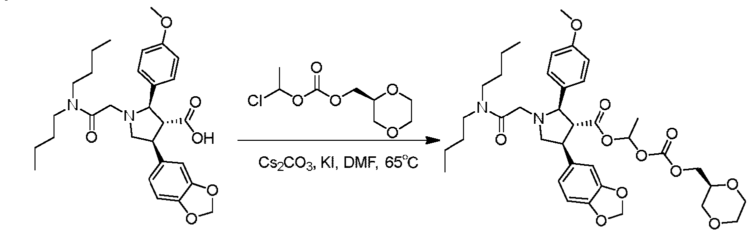

At room temperature, Atrasentan (2*R*,3*R*,4*S*)-4-(benzo[*d*][1,3]dioxolane-5-yl)-1-[2-(dibutylamino)-2-oxoethyl]-2-(4-methoxy phenyl)pyrrolidine-3-carboxylic acid (100 mg, 0.20 mmol), ((*S*)-1,4-dioxane-2-yl)methyl(1-chloroethyl)carbonate (44.8 mg, 0.3 mmol), cesium carbonate (130 mg, 0.4 mmol), and potassium iodide (66.4 mg, 0.4 mmol) were added to 5 mL of dry DMF and heated to 65°C to react for 4 hours.

After the reaction was completed, a mixture was cooled to room temperature, poured into 40 mL of an ice water solution, and extracted with dichloromethane (20 mL×3). Organic phases were combined. A mixture was washed with saturated salt water (50 mL), dried with anhydrous magnesium sulfate, filtered, and concentrated under reduced pressure to dryness. A crude compound was purified by silica-gel column chromatography (eluent: n-hexane/ethyl acetate=3/1) to obtain 92 mg of a colorless oily product 1-(((((*S*)-1,4-dioxane-2-yl)methoxy)carbonyl)oxy)ethyl-(2*R*,3*R*,4*S*)-4-(benzo[*d*][1,3]dioxola ne-5-yl)-1-[2-(dibutylamino)-2-oxoethyl]-2-(4-methoxyphenyl)pyrrolidine-3-carboxylate (yield: 65.8%).

LC-MS:RT = 2.14 min, [M+H]⁺ =699.43. ¹H NMR (400 MHz, DMSO) δ 7.24 (dd, *J* = 11.6, 8.7 Hz, 2H), 7.06 (d, *J =* 10.7 Hz, 1H), 6.91 (dd, *J =* 8.6, 2.7 Hz, 2H), 6.85-6.70 (m, 2H), 6.59-6.53 (m, 1H), 5.99 (d, *J =* 6.2 Hz, 2H), 4.09-3.97 (m, 2H), 3.75-3.65 (m, 6H), 3.56 (dd, *J =* 22.9, 9.5 Hz, 3H), 3.47-3.38 (m, 2H), 3.23 (dd, *J =* 31.0, 20.1 Hz, 5H), 2.96 (dt, *J* = 16.3, 8.0 Hz, 3H), 2.84-2.62 (m, 2H), 1.33 (t, *J* = 8.2 Hz, 3H), 1.29-1.20 (m, 3H), 1.19-1.07 (m, 3H), 0.96 (d, *J =* 7.4 Hz, 2H), 0.81 (t, *J =* 7.3 Hz, 3H), 0.71 (t, *J =* 7.3 Hz, 3H).

### Embodiment 12

Research on Compound Microsome

### (1) Experimental material

Human liver microsomes were all purchased from Rild Liver Disease Research (Shanghai) Co., Ltd..

Reagents: dimethyl sulfoxide (DMSO), acetonitrile, formic acid, and propranolol (internal standard) are all commercially available.

Instrument: Thermo Fisher Scientific LC-MS (U300 UPLC, TSQ QUANTUMN ULTRA triple quadrupole mass spectrometer).

### (2) Experimental method

A certain amount of compound was precisely weighed and dissolved in DMSO to prepare a 10 mM stock solution, and the stock solution was diluted with a diluent (ACN:H₂O=1:1) to a 100 µM working solution, and then diluted with a 0.1 M potassium phosphate buffer solution to a 3 µM administration solution for later use. 75 µL of liver microsomes were taken and added into 925 µL of a 0.1 M potassium phosphate buffer solution to be mixed uniformly to obtain a 1.5 mg/mL liver microsome suspension, and the liver microsome suspension was pre-incubated at 37°C for 10 minutes. Preparation at 0 o'clock: 15 µL of the above liver microsome suspension was taken and added into a 6 mM NADPH solution, 150 µL of a propranolol acetonitrile solution was added immediately for precipitation, and then 15 µL of the above administration solution was added and mixed uniformly for later use. Preparation of samples at 20 minutes and 60 minutes: 15 µL of the administration solution was taken, 15 µL of the liver microsome suspension and 15 µL of the 6 mM NADPH solution were added and mixed uniformly, and a mixture was incubated at 37°C for 20 minutes and 60 minutes, respectively. The preparation of the above samples was carried out using dual-pore parallel operation. When the above samples are incubated to the relevant time point, 150 µL of the propranolol acetonitrile solution was added to terminate the reaction. All the above samples were centrifuged at 4000 rpm for 5 minutes, and 100 µL of a supernatant was taken, added into 100 µL of ultrapure water and mixed uniformly for LC-MS/MS analysis. LC-MS/MS detection conditions are as follows:
Chromatographic column: Waters ACQUITYTM PREMIER HSS T3, 50*2.1 mm, 1.8 µm.

Mobile phase: water (0.1% formic acid) - acetonitrile. Perform gradient elution according to the table below.

| **Time (min)** | **Water (containing 0.1% formic acid)** | **Acetonitrile** |
|---|---|---|
| 0 | 85% | 15% |
| 0.6 | 85% | 15% |
| 1 | 20% | 80% |
| 2.3 | 20% | 80% |
| 2.31 | 85% | 15% |
| 3 | 85% | 15% |

### (3) Data processing

An initial 0 o'clock was taken as 100%, a relative residual content of the drug at each time point was calculated, and each time point of Atrasentan was taken as 100% to calculate the relative amount of transformation of the compound in the embodiment to Atrasentan. Results were shown in Table 1 and Table 2.

**Table 1. Changes of prototypes of various drugs in microsomes**

| **Species** | **Base** | **Compound** | **Time** | **Prototype remaining quantity (%)** |
|---|---|---|---|---|
| | | | **(min)** | **Mean** |
| Human | Liver microsome (+NADPH) | Atrasentan | 0 | 100.0 |
| | | | 20 | 83.3 |
| | | | 60 | 50.3 |
| | | 1 | 0 | 100.0 |
| | | | 20 | 0.5 |
| | | | 60 | 0.7 |
| | | 2 | 0 | 100.0 |
| | | | 20 | 67.8 |
| | | | 60 | 1.6 |
| | | 3 | 0 | 100.0 |
| | | | 20 | 0.04 |
| | | | 60 | 0.06 |
| | | 4 | 0 | 100.0 |
| | | | 20 | 0 |
| | | | 60 | 0 |
| | | 5 | 0 | 100.0 |
| | | | 20 | 0 |
| | | | 60 | 0 |
| | | 6 | 0 | 100.0 |
| | | | 20 | 0.0 |
| | | | 60 | 0.7 |
| | | 7 | 0 | 100.0 |
| | | | 20 | 0.10 |
| | | | 60 | 0.47 |
| | | 8 | 0 | 100.0 |
| | | | 20 | 0.04 |
| | | | 60 | 0.03 |
| | | 9 | 0 | 100.0 |
| | | | 20 | 0.1 |
| | | | 60 | 0.0 |
| | | 10 | 0 | 100.0 |
| | | | 20 | 0.0 |
| | | | 60 | 0.0 |
| | | 11 | 0 | 100.0 |
| | | | 20 | 0.5 |
| | | | 60 | 0.0 |

**Table 2. Transformation of Compound in embodiment to Atrasentan amount in microsome**

| **Species** | **Base** | **Compound** | **Time** | **Atrasentan production (%)** |
|---|---|---|---|---|
| | | | **(min)** | **Mean** |
| Human | Liver microsome (+NADPH) | 1 | 0 | 0 |
| | | | 20 | 18.6 |
| | | | 60 | 33.1 |
| | | 2 | 0 | 0 |
| | | | 20 | 54.8 |
| | | | 60 | 97.9 |
| | | 3 | 0 | 0.1 |
| | | | 20 | 113.4 |
| | | | 60 | 104.0 |
| | | 4 | 0 | 0.3 |
| | | | 20 | 73.2 |
| | | | 60 | 77.1 |
| | | 5 | 0 | 76.0 |
| | | | 20 | 73.4 |
| | | | 60 | 82.4 |
| | | 6 | 0 | 67.3 |
| | | | 20 | 61.0 |
| | | | 60 | 54.5 |
| | | 7 | 0 | 1.3 |
| | | | 20 | 115.1 |
| | | | 60 | 107.3 |
| | | 8 | 0 | 26.5 |
| | | | 20 | 29.0 |
| | | | 60 | 27.4 |
| | | 9 | 0 | 3.6 |
| | | | 20 | 36.4 |
| | | | 60 | 36.6 |
| | | 10 | 0 | 6.5 |
| | | | 20 | 42.6 |
| | | | 60 | 46.1 |
| | | 11 | 0 | 9.1 |
| | | | 20 | 55.9 |
| | | | 60 | 51.7 |

The results showed that the compound of Embodiment 3 can be rapidly metabolized in human microsomes and fully converted into Atrasentan.

### Embodiment 13

Pharmacokinetic Study of Compound in Rats

### (1) Experimental material

SD rats: male, 200-300g, purchased from Beijing Vital River Laboratory Animal Technology Co., Ltd..

Reagents: dimethyl sulfoxide (DMSO), polyethylene glycol 400 (PEG-400), normal saline, heparin, acetonitrile, formic acid, and propranolol (internal standard) are all commercially available.

Instrument: Thermo Fisher Scientific LC-MS/MS (U300 UPLC, TSQ QUANTUMN ULTRA triple quadrupole mass spectrometer).

### (2) Experimental method

The compound was weighed and dissolved in a DMSO-PEG-400-normal saline (5:60:35, v/v/v) system. After intragastric administration to rats, 200 µL of venous blood was collected in a sodium-fluoride heparinized EP tube at 15 minutes, 30 minutes, 1 hour, 2 hours, 5 hours, 7 hours, and 24 hours after administration and centrifuged at 12000 rpm for 2 minutes. Plasma was taken and frozen and preserved at -80°C for testing. A certain amount of samples for test was precisely weighed and dissolved in DMSO to 2 mg/mL as a stock solution. An appropriate amount of compound stock solution was accurately sucked, and diluted by adding acetonitrile to prepare a standard series solution. 20 µL of each of the above standard series solution was accurately sucked, 180 µL of blank plasma was added, and vortex mixing was performed to prepare plasma samples equivalent to plasma concentrations of 1 ng/mL, 3 ng/mL, 5 ng/mL, 10 ng/mL, 30 ng/mL, 100 ng/mL, 300 ng/mL, 1000 ng/mL, and 3000 ng/mL. Dual sample analysis was performed for each concentration to establish a standard curve. 30 µL of plasma was taken, 200 µL of an acetonitrile solution of internal standard propranolol (50 ng/mL) was added, vortex mixing was performed, then 100 µL of purified water was added, vortex mixing was performed again, a mixture was centrifuged at 4000 rpm for 5 minutes, and a supernatant was taken for LC-MS/MS analysis. LC-MS/MS detection conditions are as follows:
Chromatographic column: Waters ACQUITYTM PREMIER HSS T3, 50*2.1 mm, 1.8 µm.

Mobile phase: water (0.1% formic acid) - acetonitrile. Perform gradient elution according to the table below.

| **Time (min)** | **Water (containing 0.1% formic acid)** | **Acetonitrile** |
|---|---|---|
| 0 | 85% | 15% |
| 0.6 | 85% | 15% |
| 1 | 20% | 80% |
| 2.3 | 20% | 80% |
| 2.31 | 85% | 15% |
| 3 | 85% | 15% |

### (3) Data processing

After LC-MS/MS detection of blood drug concentration, WinNonlin 6.1 software was used to calculate pharmacokinetic parameters using a non compartmental model method. The results were shown in Table 3.

**Table 3: Atrasentan and pharmacokinetic parameters of Atrasentan after intragastric administration in SD rats according to Embodiment 3 and Embodiment 4**

| Spec ies | Compound | Dosage (mg/kg) Administrati on route | Tₘₐₓ(h) | Cₘₐₓ (ng/ml) | AUCₗₐₛₜ (h*ng/ml) | T_{1/2}(h) |
|---|---|---|---|---|---|---|
| SD rats | Atrasentan | 5.0, p.o | 0.58 | 877 | 2028 | 1.7 |
| | 2 | 5.0, p.o | 0.5 | 349 | 2140 | 7.0 |
| | 3 | 5.0, p.o | 1.92 | 494 | 2867 | 16.3 |
| | 4 | 5.0, p.o | 3.0 | 512 | 2637 | 5.07 |

| | | | | | | |
|---|---|---|---|---|---|---|
| Note: The administration dosage is converted to the Atrasentan amount | | | | | | |

Exposure levels of compounds in Embodiment 3 and Embodiment 4 in rats were higher than those of the same dosage of Atrasentan, and the absorption after intragastric administration was better than that of Atrasentan.

### Embodiment 14

Pharmacokinetic Study of Compound in Rats

### (1) Experimental material

SD rats: male, 200-300g, purchased from Beijing Vital River Laboratory Animal Technology Co., Ltd..

Reagents: dimethyl sulfoxide (DMSO), polyethylene glycol 400 (PEG-400), normal saline, heparin, acetonitrile, formic acid, and propranolol (internal standard) are all commercially available.

Instrument: Thermo Fisher Scientific LC-MS/MS (U300 UPLC, TSQ QUANTUMN ULTRA triple quadrupole mass spectrometer).

### (2) Experimental method

The compound was weighed and dissolved in a DMSO-PEG-400-normal saline (5:60:35, v/v/v) system. After intragastric administration to rats, 200 µL of venous blood was collected in a sodium-fluoride heparinized EP tube at 15 minutes, 30 minutes, 1 hour, 2 hours, 5 hours, 7 hours, and 24 hours after administration and centrifuged at 12000 rpm for 2 minutes. Plasma was taken and frozen and preserved at -80°C for testing. A certain amount of samples for test was precisely weighed and dissolved in DMSO to 2 mg/mL as a stock solution. An appropriate amount of compound stock solution was accurately sucked, and diluted by adding acetonitrile to prepare a standard series solution. 20 µL of each of the above standard series solution was accurately sucked, 180 µL of blank plasma was added, and vortex mixing was performed to prepare plasma samples equivalent to plasma concentrations of 1 ng/mL, 3 ng/mL, 5 ng/mL, 10 ng/mL, 30 ng/mL, 100 ng/mL, 300 ng/mL, 1000 ng/mL, and 3000 ng/mL. Dual sample analysis was performed for each concentration to establish a standard curve. 30 µL of plasma was taken, 200 µL of an acetonitrile solution of internal standard propranolol (50 ng/mL) was added, vortex mixing was performed, then 100 µL of purified water was added, vortex mixing was performed again, a mixture was centrifuged at 4000 rpm for 5 minutes, and a supernatant was taken for LC-MS/MS analysis. LC-MS/MS detection conditions are as follows:
Chromatographic column: Waters ACQUITYTM PREMIER HSS T3, 50*2.1 mm, 1.8 µm.

Mobile phase: water (0.1% formic acid) - acetonitrile. Perform gradient elution according to the table below.

| **Time (min)** | **Water (containing 0.1% formic acid)** | **Acetonitrile** |
|---|---|---|
| 0 | 85% | 15% |
| 0.6 | 85% | 15% |
| 1 | 20% | 80% |
| 2.3 | 20% | 80% |
| 2.31 | 85% | 15% |
| 3 | 85% | 15% |

### (3) Data processing

After LC-MS/MS detection of blood drug concentration, WinNonlin 6.1 software was used to calculate pharmacokinetic parameters using a non compartmental model method. The results were shown in Table 4.

**Table 4: Atrasentan and pharmacokinetic parameters of Atrasentan after intragastric administration in SD rats according to Embodiment 7**

| Species | Compound | Dosage (mg/kg), administration route | Tₘₐₓ(h) | Cₘₐₓ(ng/ml) | AUCₗₐₛₜ(h*ng/ml) | T_{1/2}(h) |
|---|---|---|---|---|---|---|
| SD rats | 7 | 5.0, p.o | 0.5 | 151 | 461 | 2.31 |

An exposure level of the compound in Embodiment 7 in rats was significantly lower than that of the same dosage of Atrasentan, and the absorption after intragastric administration was not as good as that of Atrasentan.

In combination with Embodiments 12, 13, and 14, overall, the compound in Embodiment 3 can be rapidly converted to Atrasentan both in vitro and in vivo, and the exposure level was higher at oral equimolar dosage compared to Atrasentan.

### Embodiment 15 Salt Screening Embodiment

A compound A was dissolved in different solvents such as isopropanol, acetone, and ethyl acetate, acids with the same molar ratio (as shown in the table below, a total of 17 acids) were added, after reacting for a certain time, a solution became clear, and a solid was precipitated by adding an anti-solvent and lowering the temperature.

**Table 5: Screening of 17 Acid Ligands**

| Serial number | Acidic ligand | Result | State of substance |
|---|---|---|---|
| 1 | Sulphuric acid | Forming a salt | Solid powder |
| 2 | Phosphoric acid | Forming a salt | Solid powder |
| 3 | Oxalic acid | Forming a salt | Solid powder |
| 4 | Hydrochloric acid | Forming a salt | Solid powder |
| 5 | Mucic acid | Not forming a salt | Oily substance |
| 6 | Maleic acid | Not forming a salt | Oily substance |
| 7 | Tartaric acid | Not forming a salt | Oily substance |
| 8 | Fumaric acid | Not forming a salt | Oily substance |
| 9 | Citric acid | Not forming a salt | Oily substance |
| 10 | Malic acid | Not forming a salt | Oily substance |
| 11 | Hippuric acid | Not forming a salt | Oily substance |
| 12 | Succinic acid | Not forming a salt | Oily substance |
| 13 | 1,5-Naphthalene disulfonic acid | Not forming a salt | Oily substance |
| 14 | P-toluenesulfonic acid | Not forming a salt | Oily substance |
| 15 | Methanesulfonic acid | Not forming a salt | Oily substance |
| 16 | Hydrobromic acid | Not forming a salt | Oily substance |
| 17 | Lactic acid | Not forming a salt | Oily substance |

The compound A is an oily substance that can only precipitate as a solid after forming a salt with a few acids. From the above table, it can be seen that the compound A has considerable difficulty in salt formation, with only a few acids such as sulfuric acid, phosphoric acid, oxalic acid, and hydrochloric acid being able to form the salt.

### Embodiment 16 Preparation of Oxalate

Compound A free alkali (3.5 g) was dissolved in isopropanol (10 ml), oxalic acid dihydrate (689 mg) was added, stirred at room temperature to react for 1 hour, and concentrated to obtain an oily substance, isopropyl ether (50 ml) was added under an ice bath and stirred to precipitate a solid, and the solid was stirred under an ice bath for 1 day, and filtered to obtain an oxalate solid.

Compound A free alkali (2.4 g) was reacted with oxalic acid dihydrate (46 mg) to form a salt in ethyl acetate (1 w/w). The salt was then added dropwise to isopropyl ether (15 w/w) at -5°C, stirred for 1 day, filtered, and dried to obtain a solid.

Compound A free alkali (2.2 g) was reacted with oxalic acid (70 mg) in isopropanol (2 ml) and concentrated, isopropyl acetate (0.5 ml) was added, and isopropyl ether (15 ml) was added at -15°C, stirred for 4 hours, and filtered to obtain a solid.

Nuclear magnetism data of oxalate:

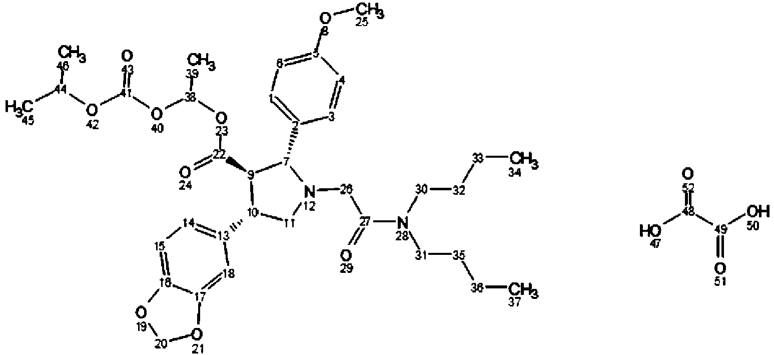

### Attribution:

| Serial number | Shift | H's | Integral | Type | J's |
|---|---|---|---|---|---|
| 1 | 0.93 | 6 | 5.89 | t | 7.94, 7.94 |
| 2 | 1.28 | 3 | 3.13 | d | 6.77 |
| 3 | 1.34 | 7 | 7.15 | m | |
| 4 | 1.52 | 4 | 4.01 | m | |
| 5 | 1.59 | 3 | 2.97 | d | 6.93 |
| 6 | 3.24 | 1 | 1.03 | m | |
| 7 | 3.29 | 1 | 1.01 | m | |
| 8 | 3.31 | 2 | 1.91 | m | |
| 9 | 3.35 | 3 | 3.02 | m | |
| 10 | 3.41 | 1 | 0.99 | dd | 6.96, 12.27 |
| 11 | 3.59 | 1 | 0.99 | dd | 6.96, 12.27 |
| 12 | 3.68 | 1 | 1.00 | qd | 0.78, 6.98, 7.00, 7.00 |
| 13 | 3.78 | 2 | 2.29 | s | |
| 14 | 4.39 | 1 | 0.95 | dd | 0.76, 6.94 |
| 15 | 5.08 | 1 | 0.99 | hept | 6.82, 6.82, 6.86, 6.86, 6.86, 6.86 |
| 16 | 5.93 | 2 | 1.96 | m | |
| 17 | 6.63 | 1 | 0.98 | dd | 0.63, 1.52 |
| 18 | 6.72 | 1 | 0.98 | d | 7.48 |
| 19 | 6.82 | 1 | 0.90 | dd | 1.43, 7.54 |
| 20 | 6.91 | 2 | 2.01 | m | |
| 21 | 7.20 | 2 | 1.87 | m | |
| 22 | 7.51 | 1 | 0.98 | Q | 6.81, 6.81, 6.85 |
| 23 | 11.05 | 2 | 2.41 | m | |

### Analysis:

a part with δ=0.93 is methyl hydrogen on C atoms 34 and 37, has the number of protons being 6, and is two groups of triplets;
a part with δ=1.28 is methyl hydrogen on C atom 45, has the number of protons being 3, and is one group of doublet;
a part with δ=1.34 is methyl hydrogen on C atom 46 and methylene hydrogen on C atoms 33 and 36, has the number of protons being 7, and is one group of triplet and two groups of doublets respectively;
a part with δ=1.52 is methylene hydrogen on C atoms 32 and 35, has the number of protons being 4, and is two groups of triplets;
a part with δ=1.59 is methyl hydrogen on C atom 39, has the number of protons being 3, and is one group of doublet;
a part with δ=3.24, 3.29, 3.31, 3.35, 3.41, 3.59, 3.68 is respectively methylene hydrogen on C atoms 30, 31, 26, 11, 9 and 10, has the number of protons being 12, and is three groups of multiplets;
a part with δ=3.78 is methyl hydrogen on C atom 25, has the number of protons being 3, and is one group of singlet;
a part with δ=4.39 is methine hydrogen on C atom 7, has the number of protons being 1, and is two groups of doublets;
a part with δ=5.08 is methine hydrogen on C atom 44, has the number of protons being 1, and is one group of multiplet;
a part with δ=5.93 is methylene hydrogen on C atom 20, has the number of protons being 2, and is one group of triplet;
a part with δ=6.63, 6.72, 6.82 is respectively methine hydrogen on C atoms 18, 15 and 14, has the number of protons being 3, and is three groups of doublets;
a part with δ=6.91 is methine hydrogen on C atoms of benzene rings 4 and 6, has the number of protons being 2, and is two groups of triplets;
a part with δ=7.20 is methine hydrogen on C atoms of benzene rings 1 and 3, has the number of protons being 2, and is two groups of triplets;
a part with δ=7.51 is methine hydrogen on C atom 38, has the number of protons being 1, and is one group of multiplet; and
a part with δ=11.05 is carboxyl hydrogen on a C atom of oxalic acid, has the number of protons being 2, and is one group of singlet.

### Embodiment 17 Preparation of Phosphate

Compound A free alkali (4.1 g) was dissolved in isopropanol (12 ml), phosphoric acid (800 mg) was added to react for 1 hour, and isopropyl ether (80 ml) was added under an ice bath and stirred for 4 hours to obtain a phosphate solid.

### Nuclear magnetism data of phosphate:

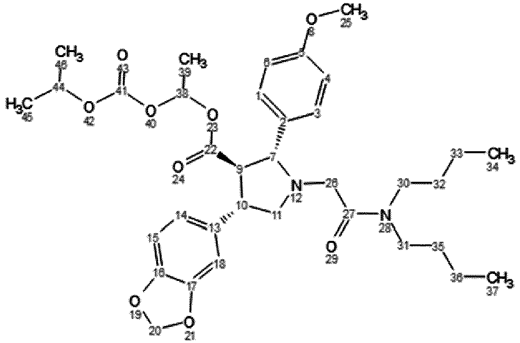

### Attribution:

| Serial number | Name | Shift | H's | Integral | Type | J's |
|---|---|---|---|---|---|---|
| 1 | Q (t) | 0.73 | 3 | 2.95 | t | 7.28, 7.28 |
| 2 | P (t) | 0.83 | 3 | 2.97 | t | 7.29, 7.29 |
| 3 | O (dd) | 0.97 | 1 | 0.95 | dd | 2.42, 7.33 |
| 4 | N (dd) | 1.01 | 1 | 0.99 | dd | 2.33, 7.85 |
| 5 | M (m) | 1.25 | 14 | 13.93 | m | |
| 6 | L (d) | 2.78 | 2 | 2.42 | d | 29.78 |
| 7 | K (dq) | 2.99 | 3 | 2.65 | dq | 5.44, 5.44, 4.09, 10.70 |
| 8 | J (td) | 3.19 | 1 | 0.63 | td | 4.65, 9.50, 9.53 |
| 9 | I (dq) | 3.31 | 2 | 2.23 | dq | 6.47, 6.47, 6.46, 14.19 |
| 10 | H (m) | 3.50 | 3 | 1.02 | m | |
| 11 | G (dp) | 4.75 | 1 | 1.03 | dp | 6.20, 6.20, 6.20, 6.20, 15.84 |
| 12 | F (dt) | 6.00 | 2 | 2.03 | dt | 1.09, 1.09, 6.09 |
| 13 | E (qd) | 6.57 | 1 | 0.98 | qd | 2.69, 5.37, 5.37, 5.37 |
| 14 | D (m) | 6.84 | 2 | 2.15 | m | |
| 15 | C (m) | 6.92 | 2 | 2.02 | m | |
| 16 | B (dd) | 7.06 | 1 | 1.02 | dd | 1.46, 13.42 |
| 17 | A (t) | 7.27 | 2 | 2.02 | t | 8.65, 8.65 |

### Analysis:

a part with δ=0.73, 0.83 is methyl hydrogen on C atoms 34 and 37, has the number of protons being 6, and is two groups of triplets;
a part with δ=1.25 is methyl hydrogen on C atoms 45, 46 and 39, methylene hydrogen on C atoms 33 and 36, and methylene hydrogen on C atoms 32 and 35, has the number of protons being 14, and is multiplets;
a part with δ=2.78, 2.99, 3.19, 3.31, 3.50 is respectively methylene hydrogen on C atoms 30, 31, 26, 11, 9 and 10, has the number of protons being 12, and is three groups of multiplets;
a part with δ=4.75 is methine hydrogen on C atom 7, has the number of protons being 1, and is two groups of doublets;
a part with δ=6.00 is methylene hydrogen on C atom 20, has the number of protons being 2, and is one group of triplet;
a part with δ=6.57, 6.84 is respectively methine hydrogen on C atoms 18, 15 and 14, has the number of protons being 3, and is three groups of doublets;
a part with δ=6.92 is methine hydrogen on C atoms of benzene rings 4 and 6, has the number of protons being 2, and is two groups of triplets; and
a part with δ=7.06, 7.27 is methine hydrogen on C atoms of benzene rings 1 and 3, and methine hydrogen on C atom 38, has the number of protons being 3, and is two groups of triplets.

Elemental analysis, P element content (%)=6.46, relative standard deviation (%)=0.2.

Detection instrument: full-spectrum direct-reading plasma atomic-emission spectrometer, iCAP6500Duo (Thermo, USA);
Detection conditions: incident power: 1150 W; plasma gas flow rate: 14 L/min; atomizer flow rate: 0.5 L/min.

### Embodiment 18 Preparation of Hydrochloride

Compound A free alkali (202.5 mg) was weighed into a 5mL vial, and MeOH (1.0 mL), and an equimolar hydrochloric acid methanol solution were added. A mixture was subjected to suspension stirring at -20°C for 1 day, methanol was removed by rotary evaporation, and a sample is in a gel form. The sample still remains the gel form when transferred to vacuum drying. Isopropyl ether (2.0 mL) was added, a mixture was subjected to suspension stirring at -20°C for 1 day, and a solid was precipitated, centrifuged and transferred to vacuum drying to obtain a solid.

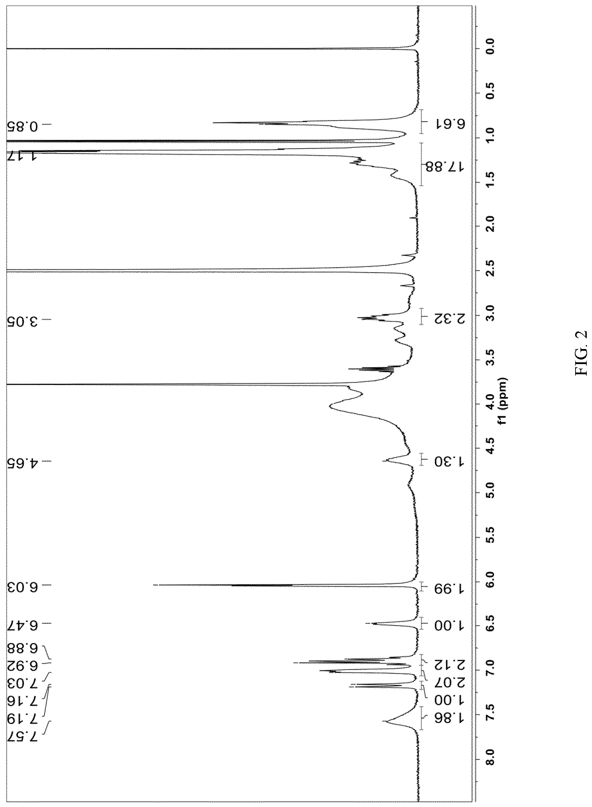

A nuclear magnetic spectrum is as shown in FIG. 2.

### Embodiment 19 Preparation of Sulphate

Compound A free alkali (203.1 mg) was weighed into a 5mL vial, an IPA (1.0 mL) solvent, and equimolar sulfuric acid were added, a mixture was subjected to suspension stirring at -20°C for 1 day, and then transferred to vacuum drying to remove IPA, and a sample is in a gel form. Isopropyl ether (2.0 mL) was added, a mixture was subjected to suspension stirring at -20°C for 1 day, and a solid was precipitated, centrifuged and subjected to vacuum drying to obtain a solid.

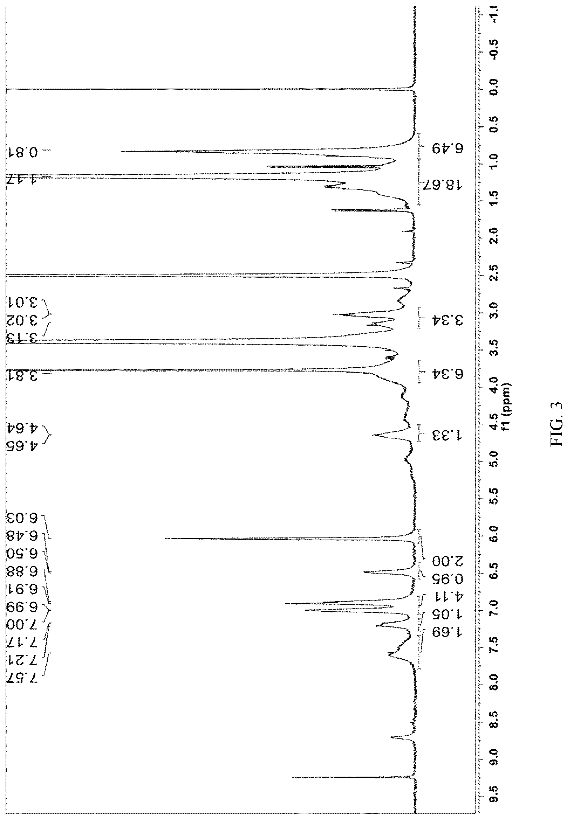

A nuclear magnetic spectrum is as shown in FIG. 3.

### Embodiment 20 Study on Stability

Sulphate, phosphate, hydrochloride, and oxalate were placed at conditions of 25°C/60%RH and 40°C/75%RH respectively for one week, and a chemical stability of samples was detected by HPLC.

| | Condition | Point in time | Purity (area%) |
|---|---|---|---|
| Sulphate | Start | - | 99.01 |
| | 25°C/60%RH | One week | 97.72 |
| | 40°C/75%RH | One week | 91.30 |
| Phosphate | Start | - | 99.29 |
| | 25°C/60%RH | One week | 98.81 |
| | 40°C/75%RH | One week | 95.49 |
| Hydrochloride | Start | - | 96.36 |
| | 25°C/60%RH | One week | 91.20 |
| | 40°C/75%RH | One week | 86.98 |
| Oxalate | Start | - | 99.56 |
| | 25°C/60%RH | One week | 99.39 |
| | 40°C/75%RH | One week | 98.52 |

From the above table, it can be seen that the oxalate and phosphate obtained by the present disclosure have good stability and are superior to hydrochloride and sulphate.

### Embodiment 21 Study on Hygroscopicity

Hygroscopicity of sulphate, phosphate, hydrochloride, and oxalate was evaluated using a dynamic vapor sorption (DVS). The test collected percentage changes in mass of samples under a constant temperature condition of 25°C with changes in humidity. DVS test results were as follows:

| | 25°C/80%RH Vapor sorption |
|---|---|
| Sulphate | 5.17% |
| Phosphate | 1.52% |
| Hydrochloride | 4.53% |
| Oxalate | 0.96% |

From the above table, it can be seen that the oxalate and phosphate obtained by the present disclosure have lower hygroscopicity and are superior to hydrochloride and sulphate.

The above embodiments are preferred implementations of the present disclosure, but the implementations of the present disclosure are not limited by the above embodiments. Any other changes, modifications, substitutions, combinations, or simplifications made without departing from the spirit and principles of the present disclosure should be equivalent substitution methods and are included in the scope of protection of the present disclosure.

## Claims

1. A salt of a compound represented by formula (I), wherein, 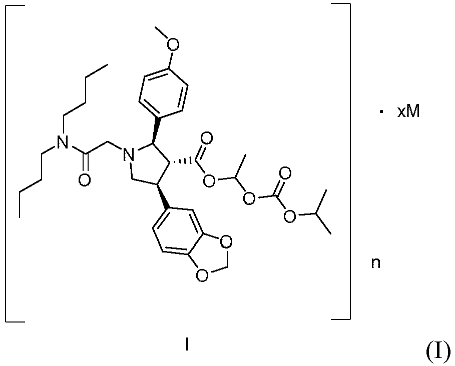 M is an inorganic acid or an organic acid, where n:x=1:(1-2).

2. The salt of the compound represented by formula (I) according to claim 1, wherein the inorganic acid is selected from the group consisting of sulfuric acid, hydrochloric acid, hydrofluoric acid, hydrobromic acid, hydroiodic acid, phosphoric acid, carbonic acid and nitric acid; and
the organic acid is selected from the group consisting of benzoic acid, 2,5-dihydroxybenzoic acid, 4-acetaminobenzoic acid, 4-aminobenzoic acid, oxalic acid, acetic acid, dichloroacetic acid, trichloroacetic acid, hexanoic acid, adipic acid, benzenesulfonic acid, 4-chlorobenzenesulfonic acid, octanoic acid, decanoic acid, cinnamic acid, citric acid, aspartate, gluconic acid, glutamic acid, lactic acid, malic acid, mandelic acid, pyroglutamic acid, tartaric acid, succinic acid, formic acid, fumaric acid, gentian acid, glutaric acid, pentanoic acid, aspartic acid, lauric acid, camphanic acid, maleic acid, propanedioic acid, nicotinic acid, propionic acid, salicylic acid, 4-aminosalicylic acid, decanedioic acid, stearic acid, butanedioic acid, palmitic acid, pamoic acid, trifluoroacetic acid, thiocyanic acid, p-toluenesulfonic acid, and L-malic acid.

3. The salt of the compound represented by formula (I) according to claim 1, wherein the inorganic acid is phosphoric acid.

4. The salt of the compound represented by formula (I) according to claim 1, wherein the inorganic acid is phosphoric acid, where n=3, x=3-6.

5. The salt of the compound represented by formula (I) according to claim 1, wherein the inorganic acid is phosphoric acid, where n=3, x=3; n=3, x=4; or n=3, x=5; or n=3, x=6.

6. The salt of the compound represented by formula (I) according to claim 1, wherein the organic acid is oxalic acid.

7. The salt of the compound represented by formula (I) according to claim 1, wherein the organic acid is oxalic acid, where n=1, x=1.

8. A method for preparing the salt of the compound according to any one of claims 1-7, wherein the salt of the compound is prepared by mixing a compound A 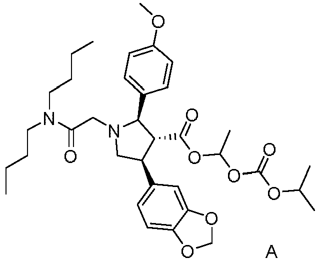 with an acid.

9. The method according to claim 8, wherein a molar ratio of the compound to an acid molecule is 1:(1-2).

10. The method according to claim 8, comprising the following steps:
1) weighing an appropriate amount of free alkali and dissolving the same with a benign solvent to obtain a solution;
2) weighing an appropriate amount of ionic acid and adding the same into the solution of step 1 for reaction to form a salt;
3) dripping a reaction solution from step 2 back into a poor solvent, stirring and precipitating; and
4) obtaining the salt of the compound by quickly centrifuging or standing.

11. A pharmaceutical composition, comprising the salt of the compound represented by formula (I) according to any one of claims 1-7, and more than one pharmaceutically acceptable carrier.

12. Use of the salt of the compound represented by formula (I) according to any one of claims 1-7 in the manufacture of a medicament for treatment and/or prevention of a disease associated with endothelin A (ETA) receptor antagonism.

13. The use according to claim 12, wherein the disease comprises chronic kidney disease, IgA, FSGS, Alport, and hypertension.
